# EUROPEAN PATENT SPECIFICATION

(11) **EP 2 679 985 B1**
(45) Date of publication and mention of the grant of the patent: **08.11.2017**
(21) Application number: 12184837.8
(22) Date of filing: 18.09.2012
(51) Int. Cl.: G01N 21/53, G01N 15/02, G01N 21/64, G01N 15/14, G01N 1/22

(54) **Real time particle fluorescence detection device**
Echtzeit-Fluoreszenzdetektionsvorrichtung
Dispositif de détection de fluorescence de particules en temps réel

(30) Priority: 28.06.2012 KR 20120069541
(43) Date of publication of application: 01.01.2014
(73) Proprietor: Agency For Defense Development, Daejeon 305-152 (KR)
(72) Inventor: Choi, Ki-Bong, 301-757 Daejeon (KR); Ha, Yeon-Chul, 302-791 Daejeon (KR); Lee, Jae-Kyung, 430-852, Gyeonggi-do (KR); Kim, Min-Cheol, Gyeonggi-do (KR)
(74) Representative: Ilgart, Jean-Christophe

(56) References cited:
- EP-A1- 1 109 005
- GB-A- 2 456 671
- JP-A- 2006 349 448
- US-A- 3 788 744
- US-A- 5 932 795
- US-B1- 6 431 014
- US-B1- 7 173 257

## Description

### [Technical Field]

The present invention relates to a device for real-time fluorescence detection of an aerosol particles, and more particularly, to a device for real-time fluorescence detection that draws into and concentrates the aerosol particles, collects the scattering light and weak fluorescence from the particle illuminated by the laser beam, separates them into several spectral fluorescence bands, and in real time detects particles (inclusive of biological aerosols) having weak fluorescent signals within an ambient environment in a rapid and reliable manner.

### [Background Art]

As the tension in international society is raised, recently, the dangers of terrorist attacks will be increased. Especially, the dangers of biological terrorism are increased because of easy manufacturing of biological weapons, easy their carrying, and easy spraying therethrough. Further, the bioterrorism causes psychological chaoses and enormous economical damages by the increment of the infection through the incubation period of pathogen.

In case where the pathogen harmful to human bodies are floating in the air, they should be detected in a short time and appropriately handled. Since they exist in the form of aerosol particles, however, they are not easily found by the naked eye. To do this, thus, a special detecting system should be needed.

Further, the pathogen are dispersed as aerosols from the front of several tens kilometers in consideration of wind direction and wind velocity, and thus, when they reach the region to be damaged, their concentration becomes substantially low. Even though the concentration is extremely low, however, the damage caused by the pathogen is not decreased at all, so that it is very important to perform the sampling and detection of both of low concentration pathogen and high concentration pathogen according to the characteristics of the atmospheric environment.

A typical method for collecting and detecting the biological aerosols such as bacteria, viruses, fungi and so on is to cultivate them on media for several hours through several days. So as to recognize the biological, that is, the sample for measurement is first collected from the air, and the amount or kind of biological aerosols in the collected sample is then measured. By the way, the above-mentioned typical method requires a lot of time and endeavors and further needs the cultivation period of several hours to several days. In this case, the sample is directly cultivated on the media, and the number of colonies formed thereon is counted to check whether the biological aerosols exists or not. However, 24 hours or more are generally needed for the cultivation of the sample, and to detect fungi, one week or more is required. Through this method, accordingly, it is impossible to perform rapid detection.

On the other hand, a laser-induced fluorescence method is widely used as a method for optically detecting the biological aerosols, and in this method, the biological aerosols are detected through the scattering light and fluorescence from the illuminated particle. However, since a light-collecting device for collecting weak fluorescence is not provided, it is hard to measure weak fluorescence.

According to conventional equipment for detecting scattering light and fluorescence by illuminating laser to biological aerosols inclusive of fluorescent non-biological aerosols, the illumination direction of the laser beam may be deviated when external vibrations or impacts are applied to the equipment, and when it is assembled again after disassembled for the interior cleaning, furthermore, the accuracy and reproducibility in the measurement of the particles may be deteriorated and the alignment work for the laser beam may be not easy.

Other examples of conventional equipment for detecting aerosols can be found in EP 1 109 005 A1, US 6,431,014 B1 and GB 2 456 671 A.

### [Disclosure]

### [Technical Problem]

Accordingly, the present invention has been made in view of the above-mentioned problems occurring in the prior art, and it is an object of the present invention to provide a device for real-time fluorescence detection that draws into and concentrates the aerosol particles, collects the scattering light and weak fluorescence from the particle illuminated by the laser beam, separates them by several fluorescence wavebands, and in real time detects particles having weak fluorescent signals contained in a rapid and reliable manner.

It is another object of the present invention to provide a device for real-time fluorescence detection that is stabilized even when external vibrations or impacts are applied thereto or when it is assembled again after disassembled, thereby improving the accuracy and reproducibility in the measurement of the fluorescent biological aerosols and easily and reliably performing the alignment of the laser beam.

### [Technical Solution]

To accomplish the above objects, according to the present invention, there is provided a device for real-time fluorescence detection adapted to separate and concentrate aerosol particles and to detect the fluorescence of the biological aerosols, the device including: a particle concentrator having a pre-separator adapted to form a passage through which the aerosol particles are drawn into and to filter foreign materials and the particles of more than the size specified from the aerosol particles, and a cylindrical casing connected to the pre-separator and having two-staged nozzles formed of a plurality of virtual impactors adapted to separate and concentrate the particles passed through the pre-separator to sizes within a given range through an inertial force; and a particle measuring unit located inside a casing assembling body disposed on the underside of the particle concentrator, characterized in that the particle measuring unit comprises: an inlet part adapted to introduce the particles and air separated and concentrated in the particle concentrator thereinto; a rectangular parallelepiped optical chamber connected to the lower end of the inlet part through an opening formed on the top surface thereof and having a particle measuring space formed at the interior thereof; a beam shaping part connected to an opening formed on the front surface of the optical chamber to illuminate laser beam to the particles introduced into the particle measuring space through the inlet part; a beam dump part connected to an opening formed on the rear surface of the optical chamber in such a manner as to face the beam shaping part to allow the laser beam L illuminated from the beam shaping part to disappear; a pair of reflectors disposed in the particle measuring space at an angle of 90° with respect to the forward direction of the laser beam; a particle discharging part connected to an opening formed on the bottom surface of the optical chamber to discharge the particles and air interacting with the laser beam in the particle measuring space to the outside through a discharging nozzle; a beam splitting part connected to an opening formed on the right surface of the optical chamber in such a manner as to be perpendicular to the beam shaping part and having a scattering light detector and fluorescence detectors adapted to detect one scattering light and two fluorescence at the same time in accordance with the cut-off frequencies of two beam splitters from the scattering light and the fluorescence signal produced by the interaction between the laser beam and the particles in the particle measuring space of the optical chamber; and the pair of reflectors comprising a spherical reflector made of a coated glass material and an aspherical reflector made of a coated aluminum material, the spherical reflector and the aspherical reflector being disposed to face each other, the back of the spherical reflector being disposed toward the beam splitting part, the spherical reflector being not coated on the center portion thereof so that the scattering light and fluorescence signals generated by illuminating the laser beam generated from the beam shaping part on the particles introduced from the inlet part are reflected to the aspherical reflector and are advanced toward the beam splitting part.

According to the present invention, preferably, the pre-separator of the particle concentrator includes: an absorption pipe to which air and aerosol particles are drawn into; a filter for removing foreign materials contained in the particles by using a mesh structure formed along the upper end periphery of the absorption pipe; a filter cap adapted to cover the top end periphery of the filter in such a manner as to allow the outer periphery thereof to be spaced apart from the filter; a funnel disposed inside the absorption pipe to filter particles having a larger size than the particles of a target size in the aerosol particles; and a cup connected to the bottom end of the funnel and having air passages formed on the side periphery thereof, into which the large particles filtered through the funnel are put.

According to the present invention, preferably, the cylindrical casing of the particle concentrator includes: the first stage nozzle formed of a plurality of virtual impactors, through which the particles within a range of the given size in the particles passed through the air passages of the cup are passed; and the second stage nozzle spaced apart from the first stage nozzle and formed of a plurality of virtual impactors, through which the particles within a range of the given size are separated and concentrated.

According to the present invention, preferably, the inlet part includes: a connection member having an open portion penetrated in an up and down direction in such a manner as to be connected to the underside end of the particle concentrator; an outside nozzle adapted to be separably coupled to the connection member in such a manner as to be fixedly connected to the top surface opening of the optical chamber at the lower end portion thereof; an insertion tube disposed at the interiors of the connection member and the outside nozzle and having an insertion hole into which the particle discharging port protrudingly extended from the underside end of the second stage nozzle is fitted; an inside nozzle fixedly connected to the interior of the insertion tube; a nozzle tip fixedly connected to the end of the outside nozzle; and an air introducing member disposed on the outside of the outside nozzle to introduce clean air from the outside thereinto.

According to the present invention, preferably, the inlet part further includes a first position determining means for determining the position of the inside nozzle when the inside nozzle is located in the connection member and the outside nozzle, the first position determining means having a concave groove formed on one side of a ring member mounted along the outer periphery of the insertion tube and a pin fixedly inserted into a pin hole formed on one side of the top periphery of the outside nozzle in such a manner as to be inserted into the concave groove.

According to the present invention, preferably, the inlet part further includes a second position determining means for determining the position of the inlet part itself when the inlet part is mounted on the optical chamber, the second position determining means having a concave groove formed on one side of a round projection formed on the outer periphery of the lower end portion of the outside nozzle and a pin disposed on one side of the opening formed on the top surface of the optical chamber in such a manner as to be inserted into the concave groove.

According to the present invention, preferably, the inlet part is fixedly connected to the top surface opening of the optical chamber by means of screw-coupling between a nut member having the upper inner periphery supported against the round projection formed on the outer periphery of the lower end portion of the outside nozzle and a screw thread formed on the inner periphery thereof and a connection member protruded upwardly from the edge of the top surface opening of the optical chamber and having a screw thread formed on the outer periphery thereof.

According to the present invention, preferably, the pin is fixedly inserted into a pin hole formed on one side of the top surface periphery of the connection member.

According to the present invention, preferably, so as to allow the inlet part to be aligned to the mounting position on the optical chamber, without any eccentricity in horizontal direction from the determined mounting position on the optical chamber when the inlet part is mounted again thereon after the separation from the optical chamber, the outside nozzle of the inlet part has an eccentricity prevention inclined portion formed on the lower end portion thereof, the eccentricity prevention inclined portion having a reversely conical structure in such a manner as to be inclined toward the top surface opening of the optical chamber, and the top surface opening of the optical chamber has an inclined surface formed on the lower end of the inner periphery thereof in such a manner as to be brought into close contact with the eccentricity prevention inclined portion.

According to the present invention, preferably, the inclined angles of the eccentricity prevention inclined portion and the inclined surface are the same as each other, and they are in a range between 25° and 35° with respect to the center axis of the inlet part.

According to the present invention, preferably, the discharging nozzle of the particle discharging part has a tapered structure being reduced in diameter toward the front end portion thereof, while being disposed to face the nozzle tip connected to the end of the outside nozzle of the inlet part in a state of being spaced apart by a given distance from the nozzle tip.

According to the present invention, preferably, the beam shaping part includes: a light source portion having a body having an inner space open at both end portions thereof to mount an aspherical lens thereinto and a cover plate having a socket in which a laser diode is embedded at the front of the body; and a beam forming and adjusting portion separably coupled to the light source portion and having a first lens group, a second lens group and a window sequentially arranged thereinto, the first lens group adapted to adjust the size in the longitudinal direction of the laser beam generated from the laser diode and passed through the aspherical lens, and the second lens group adapted to adjust the size in the transverse direction of the laser beam passed through the first lens group, and the beam shaping part is blackened on the inner surface thereof.

According to the present invention, preferably, the light source portion of the beam shaping part includes a laser beam alignment means having a plurality of hex bolts inserted through a plurality of first through taps formed spaced apart from each other on the inner edge periphery of the cover plate and a plurality of non-through taps formed on the body in such a manner as to correspond to the first through taps, each hex bolt having a spring mounted on the outer periphery thereof to adjust the pressurizing force of the cover plate toward the body, and a plurality of headless bolts inserted through a plurality of second through taps formed between the neighboring first through taps formed on the cover plate to adjust the pushing force of the cover plate from the body in accordance with the fastening degree thereof.

According to the present invention, preferably, each headless bolt is inserted into a bushing fitted to the second through tap and has a ball inserted into the end thereof to perform torque offset, the bushing having an incision groove formed on one side surface in a longitudinal direction thereof.

According to the present invention, preferably, the laser beam alignment means further includes fastening bolts inserted through three groups of third through taps spaced apart from each other along the periphery of the cover plate in such a manner as to communicate with the second through taps, to fasten and fix the bushings into which the headless bolts are inserted.

According to the present invention, preferably, each group of third through taps is formed of three through taps of a triangular shape.

According to the present invention, preferably, the light source portion of the beam shaping part further includes: a cylindrical portion formed on one side surface of the cover plate in such a manner as to be protruded horizontally toward the body and having an inner space through which the laser beam generated from the laser diode is passed; a fixing plate disposed at the inside of the front end portion of the body in such a manner as to have an open portion formed to accommodate the cylindrical portion thereinto in the state of surrounding the outer periphery of the cylindrical portion, the fixing plate being fixed to the body by means of a fastening bolt; and an aspherical lens fixing portion mounted on the inner space of the cylindrical portion and having open both end portions thereof in such a manner as to fix the aspherical lens to the inner space thereof.

According to the present invention, preferably, the cylindrical portion has a fourth through tap formed on the top surface thereof in such a manner as to communicate with the inner space thereof, the fourth through tap having a fastening screw inserted thereinto, the aspherical lens fixing portion has an alignment groove formed on the top surface of the rear end portion thereof to align the position of the aspherical lens fixing portion, and the body of the light source portion has an alignment hole formed on one side of the top surface thereof in such a manner as to communicate with the inner space of the body and to correspond to the fastening screw inserted into the fourth through tap of the cylindrical portion and the position of the alignment groove of the aspherical lens fixing portion, so as to allow the position of the center shaft of the cylindrical portion and the position of the center shaft of the aspherical lens fixing portion to be adjusted from the outside.

According to the present invention, preferably, the beam dump part includes: a body fixed to the rear surface of the optical chamber; a focal lens assembly adapted to be inserted into an insertion through-hole formed on the center of the body; a pin hole assembly fitted to a concave portion formed on the rear surface of the body in such a manner as to allow the optical axis of the focal lens assembly to be passed through a pin hole; a housing fixed to the rear end of the pin hole assembly; and a light source output detector fixed to the rear end of the housing in such a manner as to be inclined by a given angle with respect to the reference surface perpendicular to the optical axis of the focal lens assembly, the pin hole assembly and the housing having a cavity formed on the center portions thereof to allow the laser beam passed through the pin hole of the pin hole assembly to be expanded in width as it comes near to the light source output detector.

According to the present invention, preferably, the aspherical reflector is disposed on the opening formed on the left side surface of the optical chamber, and the opening is closed by a separable sealing plate.

According to the present invention, preferably, the scattering light detector has an avalanche photodiode (APD) and an amplifier.

According to the present invention, preferably, one of the fluorescence detectors is adapted to detect a long wavelength signal and the other to detect a short wavelength signal, each fluorescence detector having an optical filter mounted on the front surface thereof to cut off the scattered light and pass the induced fluorescence therethrough.

According to the present invention, preferably, the fluorescence detectors are formed of photomultiplier tubes (PMTs).

### [Advantageous Effects]

According to the present invention, only the particles of more than a given size are separated and concentrated from the the particles drawn into from the air, and then, the scattering light and weak fluorescence from the particle illuminated by the laser beam and separated into several spectral fluorescence bands, thereby in real time analyzing high concentration air state of one hundred fifteen thousand per second.

Additionally, the device for real-time fluorescence detection can be stabilized even when external vibrations or impacts are applied thereto or when it is assembled again after disassembled, thereby improving the accuracy and reproducibility in the measurement of the fluorescent biological and non-biological aerosols in the particles and easily and reliably performing the alignment of the center shaft of the inlet part mounted on the optical chamber and the laser beam.

Moreover, the size of the laser beam can be adjusted and aligned in the particle measuring space in the interior of the optical chamber where the laser beam and the particles meet each other, and especially, the spherical reflector and the aspherical reflector are disposed to face each other in the particle measuring space of the optical chamber, thereby collecting weak scattering light and fluorescent signals to a maximum degree.

Further, one-channel scattering light and two-channel biological aerosols fluorescence produced by means of the interaction in the particle measuring space of the optical chamber are measured at the same time to perform a optimization process.

Accordingly, the device for real-time fluorescence detection according to the invention can be usefully used as fluorescence detecting equipment that rapidly and reliably performs real-time monitoring for harmful biological aerosols sprayed in the air by terrorists around important facilities.

### [Description of Drawings]

FIG.1 is a perspective view showing a device for real-time fluorescence detection according to the present invention.
FIG.2 is a side sectional view showing a particle concentrator of the device for real-time fluorescence detection according to the present invention.
FIG.3 is a sectional view showing air flows in the particle concentrator.
FIGS.4a and 4b are perspective and sectional views showing a first stage nozzle of the particle concentrator.
FIGS.5a and 5b are perspective and sectional views showing a second stage nozzle of the particle concentrator.
FIG.6 is a perspective view showing an assembling state of a particle measuring unit of the device for real-time fluorescence detection according to the present invention.
FIG.7 is an exploded perspective view showing the particle measuring unit of the device for real-time fluorescence detection according to the present invention.
FIG.8 is an exploded perspective view showing an inlet part of the particle measuring unit.
FIG.9 is a sectional view showing an optical chamber.
FIG.10 is a sectional view showing the state where the inlet part is mounted on the optical chamber.
FIGS.11 to 13 are sectional views showing the particle measuring unit seen in every direction.
FIG.14 is a schematic view showing lenses arranged in a beam shaping part of the particle measuring unit of the device for real-time fluorescence detection according to the present invention.
FIG.15 is a perspective view showing a spherical reflector and an aspherical reflector located at 90° with respect to the laser beam forward direction.
FIG.16 is a sectional view showing a light source portion of the beam shaping part.
FIG.17 is a perspective view showing the fastening state of a cover plate of the light source portion.
FIG.18 is a plan view showing the cover plate of the light source portion.
FIG.19 is a partially perspective view showing the particle measuring unit of the device for real-time fluorescence detection according to the present invention.
FIG.20 is a perspective view showing the outer shape of a beam dump part.
FIG.21 is an exploded perspective view showing the beam dump part.
FIG.22 is a sectional view showing the beam dump part.
FIG.23 is a perspective view showing the operating principle of the beam dump part.
FIG.24 is an exploded perspective view showing a beam splitting part of the particle measuring unit.
FIGS.25a and 25b are sectional and rear views showing beam splitters.
FIG.26 is a perspective view showing the mounting state of the beam splitters onto a housing of the beam splitting part.

### [Mode for Invention]

Hereinafter, an explanation on a device for real-time fluorescence detection according to the present invention will be in detail given with reference to the attached drawings.

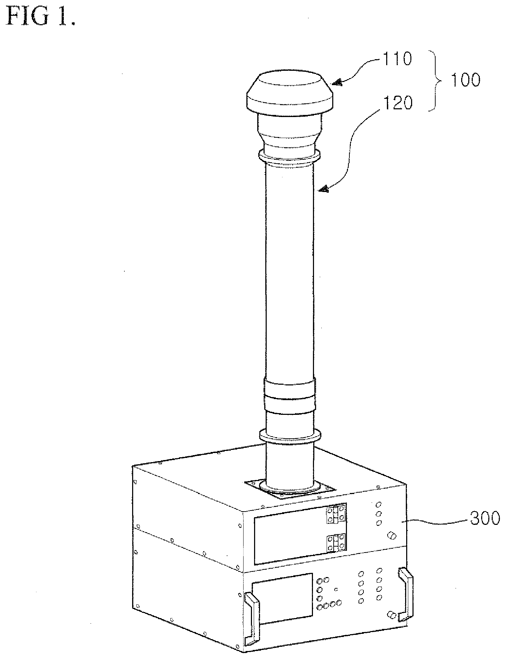

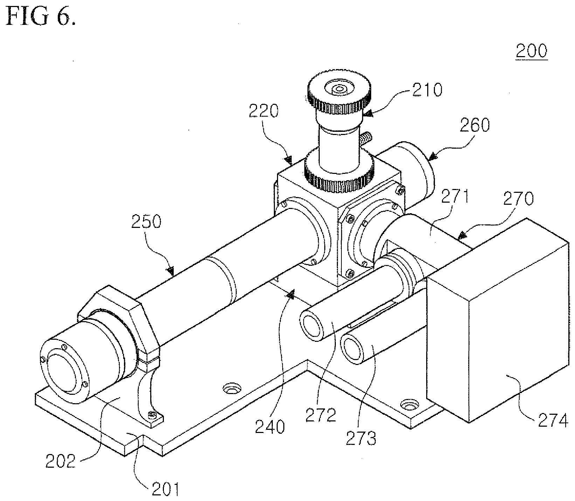

A device for real-time fluorescence detection is a device that splits scattering light and laser-induced fluorescence, which are generated when laser beam is illuminated to aerosol particles, by wavelength band, to in real time monitor the changes in the intensity of the fluorescent particles (inclusive of biological aerosols). As shown in FIG.1, the device for real-time fluorescence detection is largely composed of a particle concentrator 100 and a particle measuring unit 200 (see FIG.6) that is located inside a casing assembling body 300 disposed on the underside of the particle concentrator 100. The particle concentrator 100 and the particle measuring unit 200 are coupled separably to each other so that they can be easily cleaned and repaired when the interiors are stained and malfunctions occur.

The particle concentrator 100 is adapted to draw into and separately concentrate the aerosol particles and to convey the concentrated particles to the particle measuring unit 200, so that fine changes in the intensity of air can be in real time analyzed through the concentration of the aerosol particles with high efficiency. Generally, the aerosol particles are composed of biological aerosols and non-biological aerosols. So as to perform real-time analysis for the changes of the intensity of fluorescent particles of more than 1µm (inclusive of biological aerosols with fluorescence and some of non-biological aerosols with fluorescence), the particles of more than 1µm (especially, fluorescent particles) should be counted, but really, the number of particles having such sizes is not large, thereby making it difficult to perform real-time statistical approaches thereto. Contrarily, in places where the devices for real-time fluorescence detection are located according to the characteristics of domestic areas, mostly, a lot of pollen (of more than several hundreds µm) existing in spring seasons blows, and if foreign materials are introduced for a few days into the optical chamber as a particle measuring space during the measurement of the aerosol particles, the interior of the optical chamber becomes polluted to inconveniently need frequent cleaning. Thus, the inventor has proposed the small-sized high efficiency particle concentrator 100 capable of concentrating 100 liters per minute, which achieves the real-time approach to the quantity of variation of the fluorescent particles, that is, increases the reliability of the measurement of the changes in the intensity of the fluorescent particles. Additionally, the particle concentrator 100 filters only the particles of desired sizes, thereby preventing the pollution of the interior of the optical chamber. As mentioned above, the particle concentrator 100 serves to filter large particles and extremely fine particles.

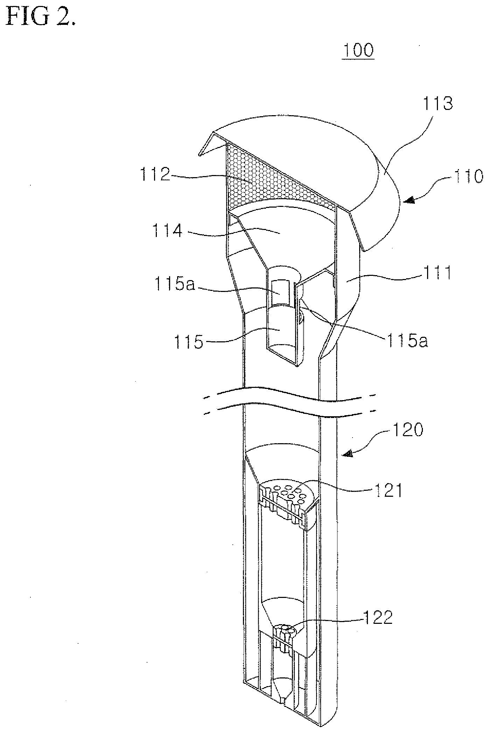

As shown in FIG.2, the particle concentrator 100 includes: a pre-separator 110 adapted to form a passage through which the particles are drawn into and to filter particles of more than a given size; and a cylindrical casing 120 having two-staged nozzles 121 and 122 formed of a plurality of virtual impactors adapted to separate and concentrate the the particles passed through the pre-separator 110 by size through an inertial force, the cylindrical casing 120 being located vertically on the top surface of the casing assembling body 300 in which the particle measuring unit 200 is accommodated in such a manner as to be connected to the pre-separator 110.

The pre-separator 110 includes an absorption pipe 111 to which air and aerosol particles are drawn into, a filter 112 for removing insects and coarse particles contained in the the particles by using a mesh structure formed along the upper end periphery of the absorption pipe 111, a filter cap 113 adapted to cover the top end periphery of the filter 112 in such a manner as to allow the outer periphery thereof to be spaced apart from the filter 112, a funnel 114 disposed inside the absorption pipe 111 to filter particles having a larger size than the particles of a target size in the aerosol particles, and a cup 115 connected to the bottom end of the funnel 114 and having air passages 115a formed on the side periphery thereof, into which the large particles filtered through the funnel 114 are put.

The cylindrical casing 120 is structured in the interior thereof to have the first stage nozzle 121 formed of a plurality of virtual impactors, through which only the particles within a range of a given size in the the particles passed through the air passages 115a of the cup 115 are passed, and to have the second stage nozzle 122 spaced apart from the first stage nozzle 121 and formed of a plurality of virtual impactors, through which only the particles within a range of the given size are separated and concentrated.

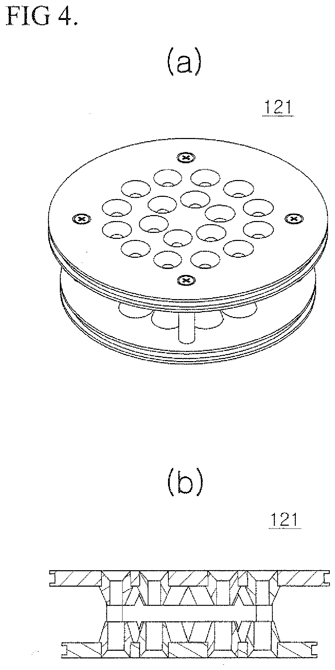

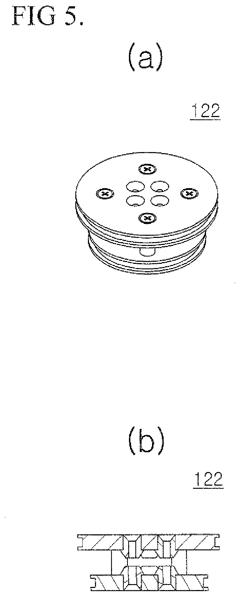

In this case, the number of virtual impactors constituting the first stage nozzle 121 is 15 through 20, and it is desirably 18 (see FIGS.4a and 4b). Further, the number of virtual impactors constituting the second stage nozzle 122 is 3 through 4, and it is desirably 4 (see FIGS.5a and 5b). Each virtual impactor makes use of a virtual pipe to collect particles in the state floating in the air, so that advantageously, the particles do not scatter. The technology on such virtual impactor has been disclosed in Korean Patent No.10-0121552, and for the brevity of the description, a detailed explanation thereof will be avoided.

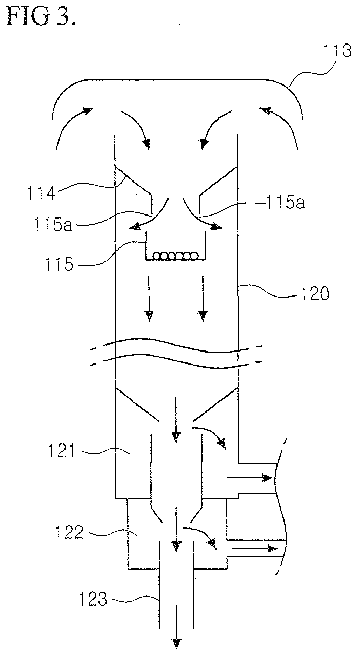

Through the installation of the two staged nozzles 121 and 122, as shown in FIG.3, if it is assumed that the flow of air passed through the air passages 115a of the cup 115 is for example, 90 liters per minute, the flow of air passed through the first stage nozzle 121 is 9 liters per minute, as the flow of concentrated air, and the rest, the flow of air of 81 liters per minute is ejected to the side. Further, the flow of air passed through the second stage nozzle 122 and then supplied to the particle measuring unit 200 disposed on the underside of the particle concentrator 100 is 1 liter per minute, as the flow of concentrated air, and the rest, the flow of air of 8 liters per minute is ejected to the side. At this time, mostly, the particles having the flow of concentrated air of 1 liter per minute passed through the second stage nozzle 122 have, for example, sizes in a range from 2µm to 10µm.

According to the particle concentrator 100 having the above-mentioned structure, the the particles are introduced together with air through the absorption pipe 111 of the pre-separator 110 by means of the operation of a flow meter (not shown) disposed on one side of the lower end of the cylindrical casing 120. At this time, foreign materials floating in the air such as carcasses of dead insects, coarse particles and the like are filtered through the filter 112. The air passed through the filter 112 is then passed through the air passages 115a of the cup 115 of the pre-separator 110, and in this process, the particles exceeding a given size, for example, the particles of more than 10µm are filtered. The filtered large-sized particles are collected in the cup 115 of the pre-separator 110, and only the particles of less than 10µm are passed through the air passages 115a of the cup 115 of the pre-separator 110 and supplied to the cylindrical casing 120.

The air passed through the air passages 115a of the cup 115 is introduced into the first stage nozzle 121 of the cylindrical casing 120, and through the 18 virtual impactors constituting the first state nozzle 121, the particles of more than 2µm are separated from the air by the inertial forces. Next, the air not passed through the virtual impactors of the first stage nozzle 121 is emitted through an outlet formed on one side of the lower end of the cylindrical casing 120. At this time, only the air of 9 liters per minute in the air of 90 liters per minute passed through the funnel 114 is passed through the first stage nozzle 121, and the rest, air of 81 liters per minute is emitted.

The air passed through the first stage nozzle 121 is concentrated by means of the four virtual impactors constituting the second stage nozzle 122 disposed below the first stage nozzle 121. Through the inertial force generated from the second stage nozzle 122, the particles of more than 2µm are separated and concentrated from the air, and the particles of less than 2µm are emitted to the outside together with the air not passed through the second stage nozzle 122, along another outlet formed on one side of the lower end of the cylindrical casing 120. The air passed through the second stage nozzle 122 is introduced together with the particles having a size between 2µm and 10µm into an inlet part 210 of the particle measuring unit 200 through a particle discharging port 123 protrudingly extended from the underside end of the second stage nozzle 122.

Through the structure of the particle concentrator 100, as mentioned above, the particles of given sizes from the aerosol particles are separately collected and concentrated.

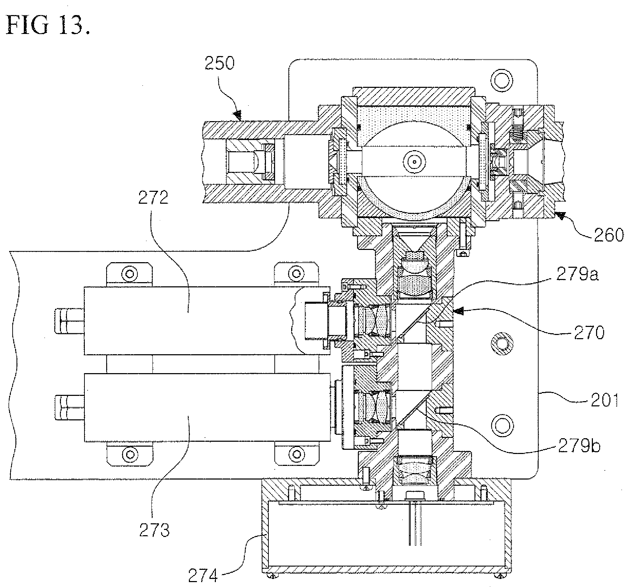

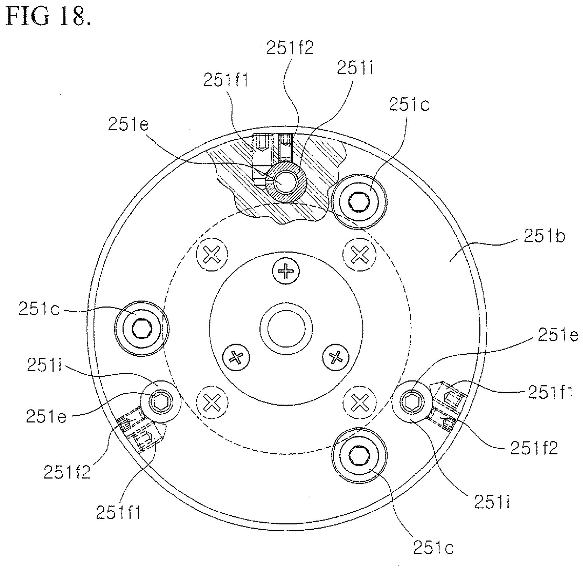

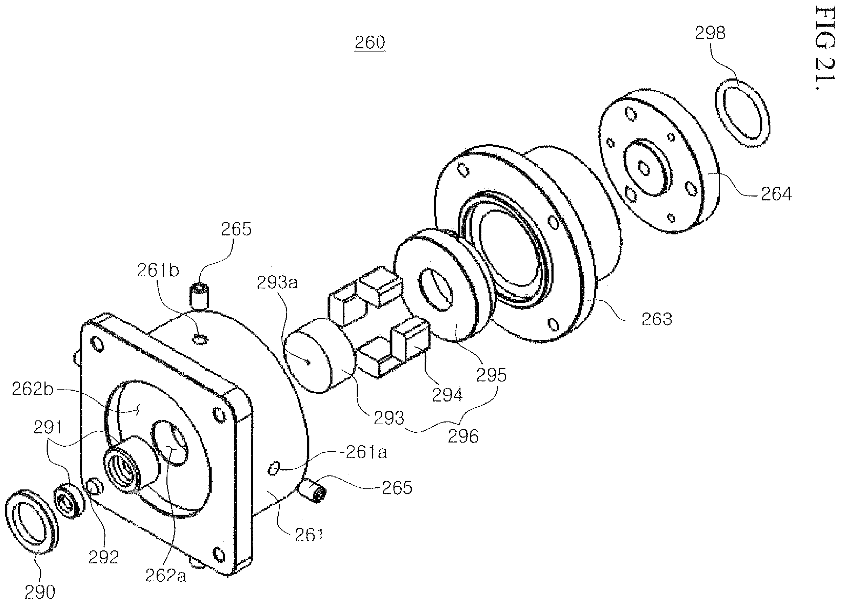

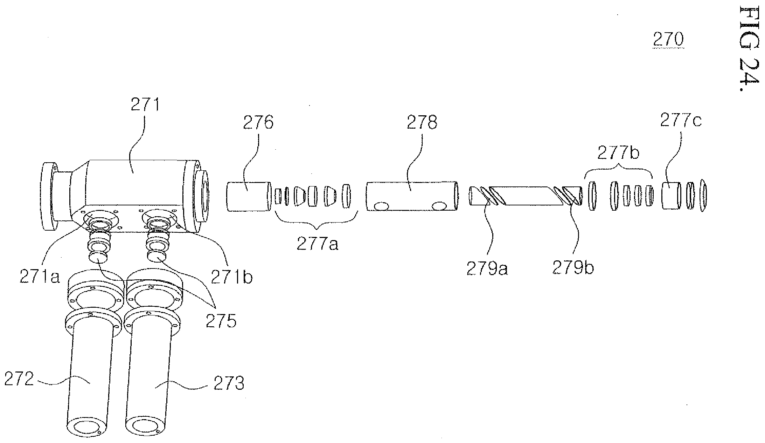

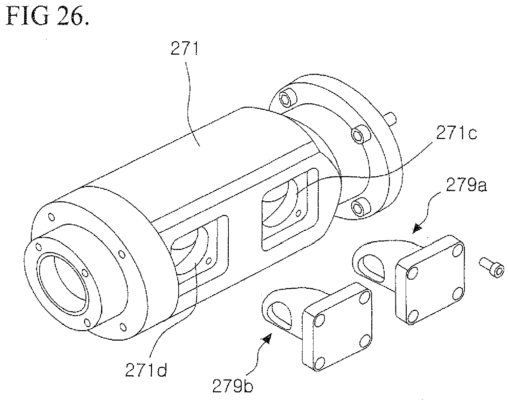

On the other hand, as shown in FIGS.6 to 26, the particle measuring unit 200 is adapted to measure laser-induced fluorescence from weak biological aerosols (inclusive of fluorescent non-biological aerosols), and it includes the inlet part 210, an optical chamber 220, reflectors 231 and 232, a particle discharging part 240, a beam shaping part 250, a beam dump part 260, and a beam splitting part 270, which are all accommodated into the casing assembling body 300 (see FIG.1).

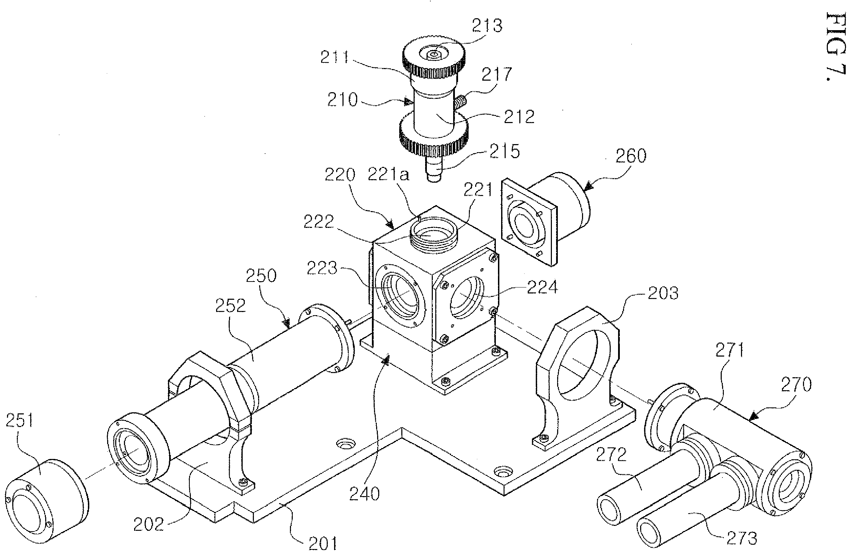

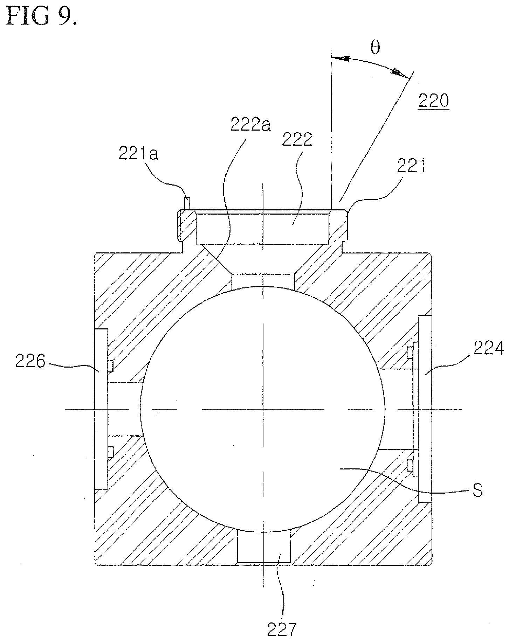

As shown in FIGS.7 and 9, the optical chamber 220 has a rectangular parallelepiped shape, into which a particle measuring space S is formed, and it also has openings 222, 223, 224, 225, 226 and 227 formed on the respective faces.

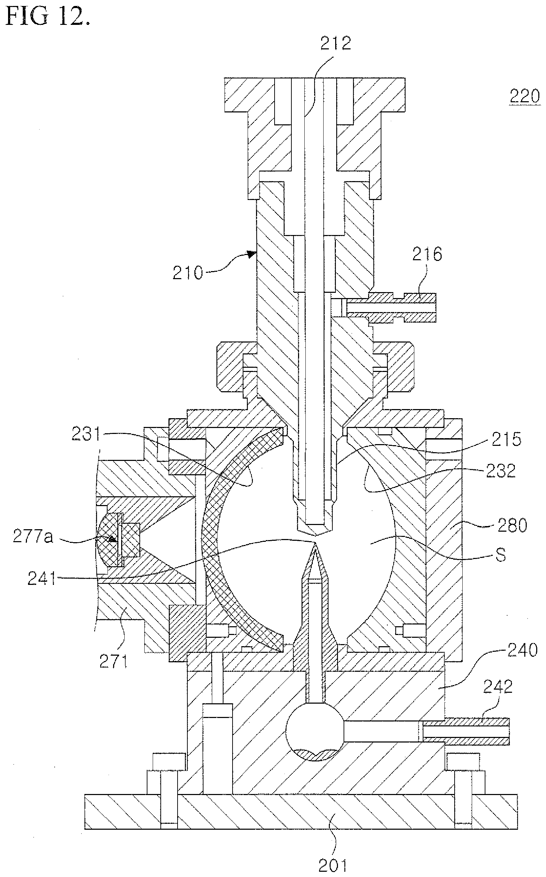

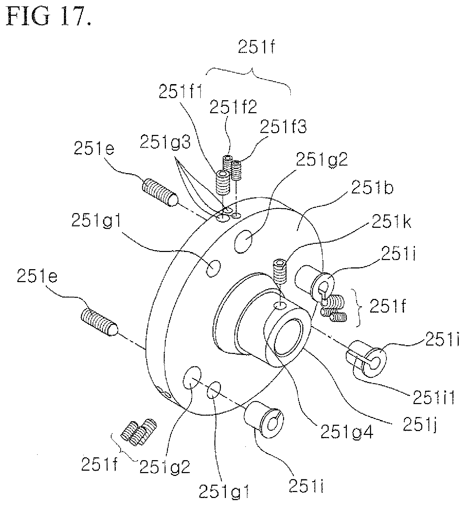

Through the openings 222, 223, 224, 225, 226 and 227, the inlet part 210 and the particle discharging part 240 are disposed on the top and underside surfaces of the optical chamber 220, and the beam shaping part 250 and the beam dump part 260 are disposed on the front and rear surfaces of the optical chamber 220 in such a manner as to face each other. Further, the beam splitting part 270 is disposed on the right side surface of the optical chamber 220. The opening 226 (see FIG.9) formed on the left side surface of the optical chamber 220 is closed by a sealing plate 280 (see FIGS.12 and 17). All of the openings of the optical chamber 220 have O-rings (not shown) mounted thereon to provide air tightness, and the inside faces of the optical chamber 220 are coated with black to efficiently draw into stray light.

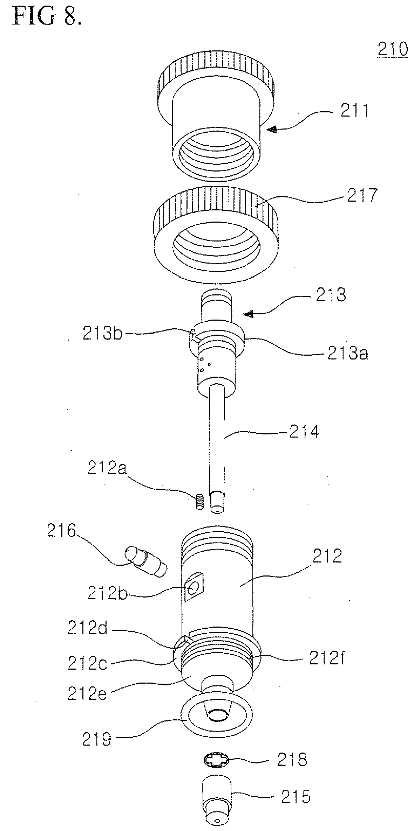

The inlet part 210 is connected to the underside end of the particle concentrator 100 to allow the particles and air separated and concentrated in the particle concentrator 100 to be introduced thereinto. That is, as shown in FIG.8, the inlet part 210 includes: a connection member 211 having an open portion penetrated in an up and down direction in such a manner as to be connected to the underside end of the particle concentrator 100; an outside nozzle 212 adapted to be separably coupled to the connection member 211 in such a manner as to be fixedly connected to the top surface opening 222 of the optical chamber 220 at the lower end portion thereof; an insertion tube 213 disposed at the interiors of the connection member 211 and the outside nozzle 212 and having an insertion hole into which the particle discharging port 123 protrudingly extended from the underside end of the second stage nozzle 122 is fitted; an inside nozzle 214 fixedly connected to the interior of the insertion tube 213; and a nozzle tip 215 fixedly connected to the end of the outside nozzle 212. Further, the insertion tube 213 has a ring member 213a mounted along the outer periphery thereof in such a manner as to be protruded therefrom in a state of encompassing the outer periphery thereof, and the ring member 213a has a concave groove 213b formed on one side thereof. The outside nozzle 212 has a pin hole (not shown) formed on one side of the top periphery thereof, and a pin 212a is fixedly inserted into the pin hole. The concave groove 213b and the pin 212a serve as a first position determining means for determining the position of the inside nozzle 214. That is, if the inside nozzle 214 is positioned in the connection member 211 and the outside nozzle 212, the pin 212a disposed on the top periphery of the outside nozzle 212 is inserted into the concave groove 213b formed on the ring member 213a of the insertion tube 213 fixedly connected to the inside nozzle 214, thereby determining the position of the inside nozzle 214.

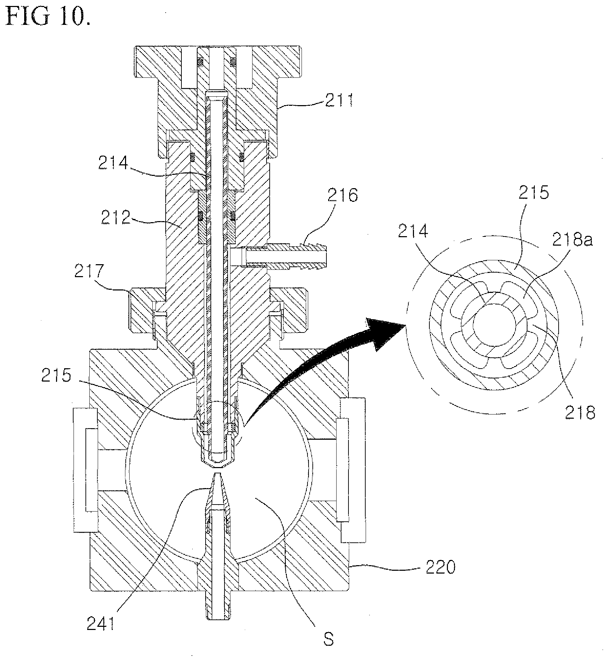

Further, the outside nozzle 212 has a fitting hole 212b formed on one side of the outer periphery of the upper end portion thereof, into which an air introducing member 216 is fitted to introduce clean air from the outside. Also, the outside nozzle 212 has a round projection 212c formed on the outer periphery of the lower end portion thereof, and the round projection 212c has a concave groove 212d formed on one side thereof. Under the above-mentioned structure, the inlet part 210 is fixedly connected to the top surface opening 222 of the optical chamber 220 by means of a nut member 217 having a screw thread formed on the inner periphery thereof in such a manner as to be fastened to the outer periphery of the lower end portion of the outside nozzle 212. That is, as shown in FIGS.7 and 9, the optical chamber 220 has a connection member 221 protruded upwardly from the edge of the top surface opening 222 in such a manner as to have a screw thread formed on the outer periphery thereof, and a pin 221a is fixedly inserted into a pin hole formed on one side of the top surface periphery of the connection member 221, so that when the lower end portion of the outside nozzle 212 of the inlet part 210 is inserted into the top surface opening 222 of the optical chamber 220, the pin 221a is first inserted into the concave groove 212d of the round projection 212c formed on the outer periphery of the outside nozzle 212, and then, the nut member 217, which is passed through the outer periphery of the connection member 211 of the inlet part 210 and inserted into the outer periphery of the outside nozzle 212 in such a manner as to allow the inner periphery of the top portion thereof to be supportedly locked to the round projection 212c, is screw-coupled to the screw thread formed on the outer periphery of the top surface opening 222 of the optical chamber 220, thereby permitting the inlet part 210 to be fixedly connected to the optical chamber 220 in a reliable manner (see FIG.10). In this case, the concave groove 212d of the round projection 212c and the pin 221a of the connection member 221 of the optical chamber 220 serve as a second position determining means for determining the positions of mounting the inlet part 210 on the optical chamber 220. Further, the nozzle tip 225 is forcedly fitted to the end of the outside nozzle 212, and at this time, the inside nozzle 214 is supported into the outside nozzle 212 by means of a snap ring 218, so that the end of the inside nozzle 214 and the end of the nozzle tip 215 are spaced apart from each other by a given gap. That is, as shown in the enlarged dotted circle of FIG.10, the outer peripheral surface of the snap ring 218 abuts against the inner peripheral surface of the outside nozzle 212, and the inner peripheral surface thereof abuts against the outer peripheral surface of the inside nozzle 214. So as to allow the clean air introduced from the air introducing member 216 of the outside nozzle 212 to be passed through the nozzle tip 215, however, the snap ring 218 has a plurality of equally spaced air passages 218a formed on the inner peripheral surface thereof. Under the above structure, the particles and air introduced into the inside nozzle 214 and the clean air introduced into the outside nozzle 212 through the air introducing member 216 of the outside nozzle 212 are collected into the gap between the end of the inside nozzle 214 and the end of the nozzle tip 215, just before they are injected into the particle measuring space S of the optical chamber 220 through the nozzle tip 215. A reference numeral 219 not explained yet denotes an O-ring fitted to a groove 212f formed along the outer periphery of the lower end portion of the outside nozzle 212.

On the other hand, the inlet part 210 is mounted again onto the optical chamber 220 after separated therefrom to clean the interior of the optical chamber 220, and thus, so as to decrease the reduction of the accuracy of the particle size measurement generated in accordance with the mounting or demounting of the inlet part 210, an eccentricity prevention inclined portion 212e is formed on the lower end portion of the outside nozzle 212, that is, between the groove 212f into which the O-ring 219 is fitted and the end of the outside nozzle 212 into which the nozzle tip 215 is fitted, having a reversely conical structure in such a manner as to be inclined toward the top surface opening 222 of the optical chamber 220. As shown in FIG. 9, the top surface opening 222 of the optical chamber 220 has an inclined surface 222a formed on the lower end of the inner periphery thereof to correspond to the eccentricity prevention inclined portion 212e. Now, the structures of the eccentricity prevention inclined portion 212e and the inclined surface 222a will be in detail described. First, if it is desired to clean the inlet part 210 or the optical chamber 220 due to the extreme environmental conditions like yellow sand, the inlet part 210 should be mounted again onto the optical chamber 220 after separated therefrom. At this time, the lower end portion of the inlet part 210 has to be mounted accurately with clearance of several tens µm at a set position of the particle measuring space S in the optical chamber 220. If the inlet part 210 is not mounted accurately onto the top surface opening 222 of the optical chamber 220, that is, if the center axis of the outside nozzle 212 to be positioned into the particle measuring space S is deviated from the original mounting position in the optical chamber 220, the center axis of the outside nozzle 212 does not cross the laser beam of several hundreds µm, and even though it crosses the laser beam, it does not exist at the accurate position, which gives bad influences on the measurement of the sizes of the particles and the measurement of the intensity of the fluorescence. By the way, it is not easy to improve the accuracy in the optical particle counter through which the particles are measured. So as to solve the above-mentioned problems, as shown in FIG.8, the invention adopts the structures wherein the pin 212a is inserted into the pin hole formed on the top surface of the outside nozzle 212 of the inlet part 210 and the concave groove 213b into which the pin 212a is inserted is formed on the insertion tube 213 connected to the inside nozzle 214, wherein the pin 221a is inserted into the pin hole formed on the connection member 221 of the optical chamber 220 and the concave groove 212d into which the pin 221a is inserted is formed on the round projection 212c formed on the outer periphery of the outside nozzle 212 of the inlet part 210, and wherein the contacted surfaces between the lower end portion of the outside nozzle 212 of the inlet part 210 and the top surface opening 222 of the optical chamber 220 have reverse conical shapes.

Under the above-mentioned structures, when the inlet part 210 separated from the optical chamber 220 is mounted again onto the optical chamber 220, the inside nozzle 214 is mounted in such a manner as to allow the pin 212a provided above the outside nozzle 212 to be fitted to the concave groove 213b of the insertion tube 213 connected thereto, thereby preventing the center axis of the inside nozzle 214 from being misaligned. The connection member 211 and the outside nozzle 212 are mounted onto the optical chamber 220 in such a manner as to allow the concave groove 212d of the round projection 212c formed on the outer periphery of the outside nozzle 212 to be aligned to the pin 221a provided on the connection member 221 of the optical chamber 220, thereby preventing the inlet part 210 itself from being misaligned. Further, the eccentricity prevention inclined portion 212e of the outside nozzle 212 is brought into close contact with the inclined surface 222a of the top surface opening 222 of the optical chamber 220, so that the center axis of the inlet part 210 is aligned to a set position of the optical chamber 220, without having any eccentricity in a horizontal direction. According to the above-mentioned structures, moreover, there is another effect that the device is stabilized against the vibrations generated by outside impacts. According to the present invention, the inclined angles of the eccentricity prevention inclined portion 212e and the inclined surface 222a are the same as each other, and they are in a range of 30.0° ± 5.0° (that is, between 25° and 35°) with respect to the center axis of the inlet part 210. Therefore, even though the inlet part 210 is mounted repeatedly onto the optical chamber 220 after separated therefrom for the cleaning work, it can be reliably mounted on the optical chamber 220 through the coupling structures between the pin 212a and the concave groove 213b and between the eccentricity prevention inclined portion 212e and the inclined surface 222a.

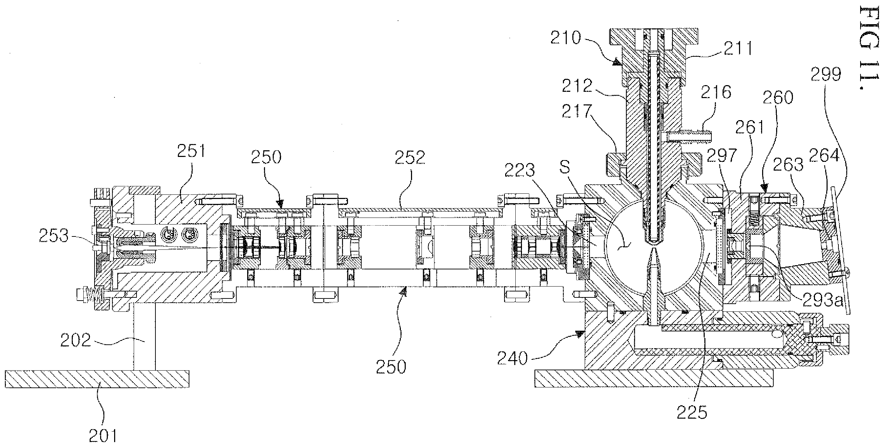

Under the above-mentioned configuration of the inlet part 210, the particles separated and concentrated in the particle concentrator 100 and a given flow of air are passed sequentially through the insertion tube 213, the inside nozzle 214 and the outside nozzle 212 of the inlet part 210 and then discharged to the particle measuring space S (see FIG.11) of the optical chamber 220. When discharged, desirably, clean air is introduced from the air introducing member 216 mounted on the outside nozzle 212 of the inlet part 210. Like this, the clean air introduced into the outside nozzle 212 through the air introducing member 216 meets the air flowing through the inside nozzle 214 in the gap (approximately 1.3mm) between the inside nozzle 214 and the outside nozzle 212 and is then passed through the nozzle tip 215. At this time, the clean air introduced into the outside nozzle 212 is injected toward a discharging nozzle 241 in the optical chamber 220 through the nozzle tip 215, while encompassing the air existing into the inside nozzle 214. As a result, the air concentrated in the minute gap can be passed, without giving any pollution to the reflectors 231 and 232 in the optical chamber 220, thereby preventing the fine particles in the air introduced through the top end of the inlet part 210 from being attached to the inner surface of the optical chamber 220 to cause the pollution of the optical chamber 220.

In the state where the inlet part 210 is mounted onto the optical chamber 220, the particles introduced into the optical chamber 220 through the top end of the inlet part 210 interact with the laser beam L in the particle measuring space S of the optical chamber 220 and are then drawn into through the discharging nozzle 241 disposed in the particle discharging part 240. Next, the particles are passed through a filter and then discharged to the outside.

In the particle measuring space S inside the optical chamber 220, a pair of reflectors 230 is disposed to collect weak scattering light and fluorescent signals and to reflect them to the beam splitting part 270. As shown in FIGS.12 to 15, the pair of reflectors 230 is composed of the spherical reflector 231 and the aspherical reflector 232 disposed to face the spherical reflector 231, and they are located at an angle of 90° with respect to the forward direction of the laser beam L. Through the arrangement, the weak side scattering light and fluorescence are collected to a maximum degree to enhance signal to noise ratio SNR.

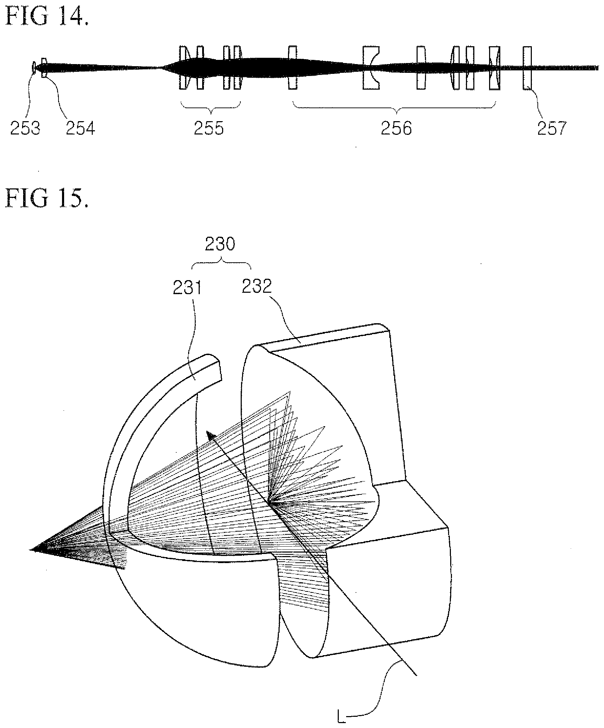

The spherical reflector 231 is made of glass, and the aspherical reflector 232 is made by attaching a reflector made of glass to a structure made of aluminum by means of an adhesive. The aspherical reflector 232 has an O-ring interposed between the aluminum structure and the glass reflector. Especially, the surfaces of the two reflectors 231 and 232 are coated to prevent the damages caused by the impurities or ultraviolet light introduced through the inside nozzle 214 and the nozzle tip 215 of the inlet part 210. In this case, however, the spherical reflector 231 is not coated on the center portion thereof, so that the scattering light and fluorescence signals generated by illuminating the laser beam L generated from the beam shaping part 250 to the particles introduced from the inlet part 210 are reflected to the aspherical reflector 232 and are advanced toward the beam splitting part 270 (see FIG.15). The formation of the spherical reflector 231 provides some advantages in easy workability of glass and the increment of the coated surface on which the weak signals are collected.

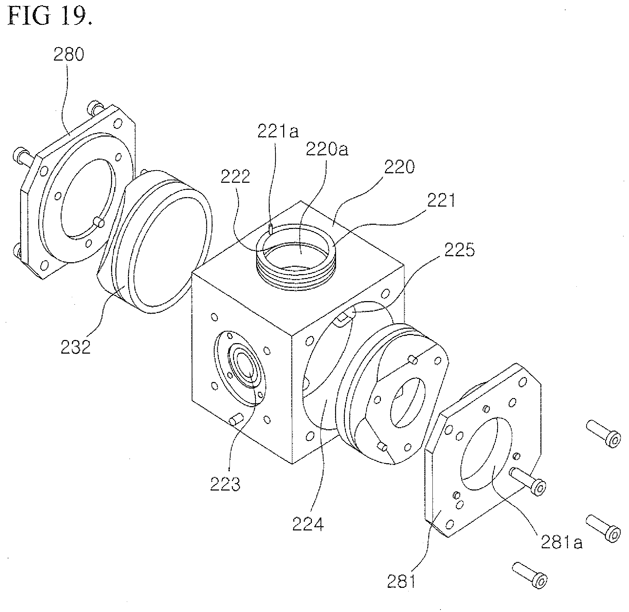

As shown in FIG.19, the spherical reflector 231 is detachably mounted on a fixing plate 281 having a through-hole 281a formed at the center thereof, and the fixing plate 281 is fastened to the right side opening 224 of the optical chamber 220 by means of fastening means like bolts.

For the easy cleaning of the optical chamber 220, as shown in FIG.12, the aspherical reflector 232 is fixed by the sealing plate 280 disposed in the optical chamber 220 in such a manner as to be detachably mounted on the outside thereof.

As shown in FIG.12, the particle discharging part 240 is disposed on the underside of the optical chamber 220 and includes the discharging nozzle 241 disposed to face the nozzle tip 215 connected to the end of the outside nozzle 212 of the inlet part 210 by a given distance and an exhaust port 242 connected to the discharging nozzle 241. The particle discharging part 240 draws into the fine particles and air introduced into the particle measuring space S of the optical chamber 220 through the outside nozzle 212 of the inlet part 210 and discharges them to the exhaust port 242. In this case, the discharging nozzle 241 of the particle discharging part 240 is tapered toward the front end thereof, thereby allowing the laser beam L to be passed therethrough and decreasing an area of the interference of the produced signal.

As shown in FIGS.6, 7, 11 and 14, the beam shaping part 250 includes a light source portion 251 having a laser diode 253 used as a light source and an aspherical lens 254 mounted thereon and a beam forming and adjusting portion 252 separably coupled to the light source portion 251 and having a plurality of lens groups 255 and 256 and a window 257 sequentially arranged thereinto, as shown in FIG.14. The beam shaping part 250 is connected to the optical chamber 220 in a state of being supported horizontally at the underside thereof through the through-hole of a vertical support rod 202 located fixedly on a support plate 201.

The beam shaping part 250 is blackened on the inner surface so as to draw into noise signals, and the parts such as the aspherical lens 254 of the light source portion 251 and the lens groups 255 and 256 and the window 257 of the beam forming and adjusting portion 252 are treated with anti-reflection coating having a reflection ratio of less than 0.26% so as to minimize the loss of the laser output. Even though not shown, a peltier element may be mounted on the outside of the light source portion 251 so as to maintain the light source portion 251 at a constant temperature therearound to decrease the output variations of the laser beam caused by the changes of the outside temperature.

As described above, since the beam forming and adjusting part 252 is separably coupled to the light source portion 251, the arrangement positions of the lens groups 255 and 256 are adjusted from the outside, thereby appropriately controlling the shape of the laser beam generated from the light source portion 251. In this case, the aspherical lens 254 of the light source portion 251 serves to enlarge the laser beam generated from the laser diode 253 with a magnifying power of 10, and the first lens group 255 of the beam forming and adjusting portion 252 serves as a beam size adjusting portion that enlarges the laser beam in the direction of x (in the transverse direction) with a magnifying power of 20 to 80, while the second lens group 256 of the beam forming and adjusting portion 252 serves as an aspect ratio adjusting portion that enlarges the laser beam in the direction of y (in the longitudinal direction) with a magnifying power of 10 to 20. Through such arrangements of the lenses, the adjustment of the size of the laser beam in the longitudinal and transverse directions in the particle measuring space S inside the optical chamber 220 can be freely performed. Like this, after the magnification of the laser beam is enlarged through the aspherical lens 254, the size of the enlarged beam is adjusted longitudinally and transversely, so that even though the production of the laser diode during the development or after the development stops or variants thereof are produced, the same performance can be achieved only through the aligning work of the laser beam axis as will be discussed later.

On the other hand, in case where a vehicle on which the device for real-time fluorescence detection is mounted is moved in mountainous regions, since the light source portion 251 in which the laser diode 253 is mounted is sensitive to weak vibrations caused from external impacts, there is a need to frequently perform optical alignment. With the rapid development of technology, further, the specifications of the laser diode during the development or after the development are varied to inconveniently cause the light source portion to be entirely changed.

In addition, when the beam shaping part 250 and the light source portion 251 are misaligned due to vibrations or when their interiors are polluted by dust, the output of the laser beam generated from the laser diode 253 becomes weak, and in this case, the accuracy of the measurement of the particle sizes becomes lowered. Especially, the UV or short wavelength laser beam acts with various dust and generally sticks to the lenses, and accordingly, even though the output of the laser beam is not varied greatly due to dust, the laser beam is hidden by the plurality of lenses polluted by dust and thus decreased in the output from the final end thereof, so that the accuracy of the measurement of the particle sizes becomes deteriorated. This problem is realized through the comparison between the output of the laser diode itself and the output of the photodiode of the beam dump part 260 as will be explained. This is very important in the maintenance of the invention, and according to the present invention, the photodiode senses the output at the final end of the laser beam, so that if the axis of the laser beam is misaligned or if the variation of the output exceeds a limitation value, an abnormal signal is just sent to a user, thereby making the axis alignment of the laser beam performed.

According to the present invention, that is, the light source portion 251 has the same performance just through the alignment of the axis of the laser beam, without having any alignment of the whole light source portion 251.

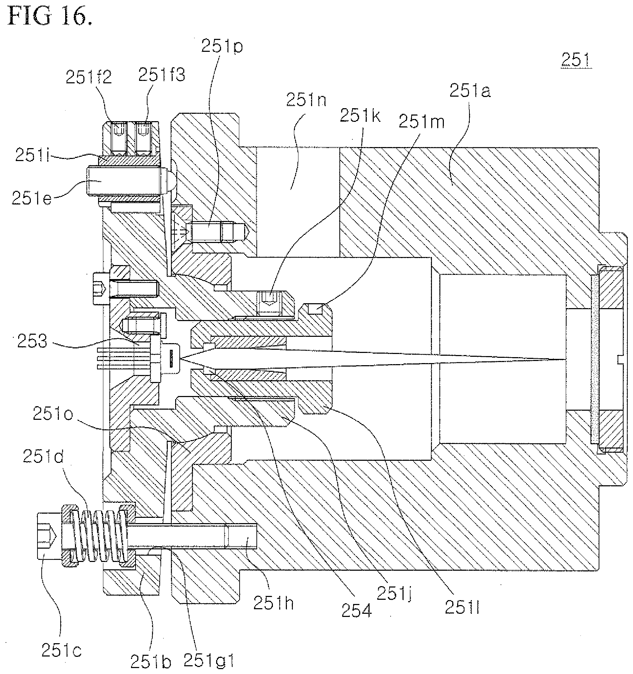

Hereinafter, the light source portion 251 will be explained with reference to FIGS.16 to 18.

As shown in FIGS.16 to 18, the light source portion 251 has a body 251a and a cover plate 251b having a socket in which the laser diode 253 is embedded at the front of the body 251a. The body 251a and the cover plate 251b are fixed to each other by means of three hex bolts 251c, and each hex bolt 251c has a spring 251d mounted on the outer periphery thereof to perform gentle adjustment, so that the cover plate 251b is movable relative to the body 251a. The three hex bolts 251c are inserted through three first through taps 251g1 formed spaced apart from each other on the inner edge periphery of the cover plate 251b and three non-through taps 251h formed on the body 251a. The first through taps 251g1 are formed in through-holes in such a manner as to allow the hex bolts 251c to be inserted thereinto and drawn therefrom, and the non-through taps 251h are formed in grooves in such a manner as to allow the screw portions of the hex bolts 251c to be inserted thereinto. A second through tap 251g2 is formed between the neighboring first through taps 251g1 formed on the cover plate 251b, and headless bolts 251e are inserted through the second through taps 251g2. The headless bolt 251e is formed of a bolt having a ball inserted into the end thereof to perform torque offset, and instead of the headless bolt 251e, a headless screw may be adopted. The headless bolt 251e is inserted into a bushing 251i fitted to the second through tap 251g2. The bushing 251i has an incision groove 251i1 formed on one side surface in a longitudinal direction thereof, as shown in FIG.16.

If the hex bolts 251c are inserted and fastened through the first through taps 251g1 and the non-through taps 251h, the cover plate 251b pressurizes the body 251a with the forces applied from the springs 251d and comes into close contact with the body 251a, thereby spatially adjusting the illumination direction of the laser diode 253. The pressurizing force of the cover plate 251b against the body 251a is varied in accordance with the fastening degrees of the hex bolts 251c. Contrarily, if the headless bolts 251e are inserted and fastened, the cover plate 251b is pushed from the body 251a, and in the same manner as above, the illumination direction of the laser diode 253 can be spatially adjusted. If the adjustment is finished, fastening bolts 251f1, 251f2 and 251f3 are inserted into three groups of third through taps 251g3 spaced apart from each other along the periphery of the cover plate 251a in such a manner as to communicate with the second through taps 251g2, and then, the fastening bolts 251f1, 251f2 and 251f3 fasten and fix the headless bolts 251e on the lateral sides. In this case, each group of third through taps 251g3 is formed of three through taps of a triangular shape. The fixation of the headless bolts 251e by the fastening bolts 251f1, 251f2 and 251f3 is carried out by inserting the fastening bolt 251f1 positioned toward the incision groove 251i of the bushing 251i into one of the third through taps 251g3 in such a manner as to allow the inner periphery of the bushing 251i to be contacted with the outer periphery of the headless bolt 251e and to fasten the incision groove 251i1 of the bushing 251i into which the headless bolt 251e is inserted and by inserting the fastening bolts 251f2 and 251f3 into the two third through taps 251g3 in such a manner as to fasten the outer periphery of the bushing 251i. If the above-mentioned works are all finished, the movement of the cover plate 251b from the body 251a of the light source portion 251 can be reliably prevented even under the application of strong vibrations caused by external impacts.

On the other hand, a cylindrical portion 251j is formed on one side surface of the cover plate 251b in such a manner as to be protruded horizontally toward the body 251a, and the cylindrical portion 251j has an inner space through which laser beam generated from the laser diode 253 is passed. The cylindrical portion 251j has a fourth through tap 251g4 formed on the top surface thereof in such a manner as to communicate with the inner space thereof, and a fastening screw 251k is inserted through the fourth through tap 251g4. The aspherical lens 254 adapted to enlarge the laser beam is mounted in the inner space of the cylindrical portion 251j in such a manner as to be fixed by an aspherical lens fixing portion 2511 insertedly fixed to the inner space of the cylindrical portion 251j, thereby preventing the movements thereof. The aspherical lens fixing portion 2511 has a cylindrical structure whose both end portions are open to allow the laser beam to be passed through the aspherical lens 254 and has an enlarged stepped portion formed at the rear end portion thereof in such a manner as to be protruded from the rear end portion of the cylindrical portion 251j. The aspherical lens 254 is located at the inside of the front end portion of the aspherical lens fixing portion 2511. An alignment groove 251m is formed on the top surface of the rear end portion of the aspherical lens fixing portion 2511, that is, the enlarged stepped portion thereof, so as to align the position of the aspherical lens fixing portion 2511.

The body 251a of the light source portion 251 has an inner space open at both end portions thereof, and the cylindrical portion 251j and the aspherical lens fixing portion 2511 are located on the inside of the front end portion of the body 251a. A fixing plate 251o is disposed at the inside of the front end portion of the body 251a in such a manner as to have an open portion formed to accommodate the cylindrical portion 251j thereinto in the state of surrounding the outer periphery of the cylindrical portion 251j of the cover plate 251b, and the fixing plate 251o is fixed to the body 251a by means of a fastening bolt 251p. Further, the body 251a of the light source portion 251 has an alignment hole 251n formed on one side of the top surface thereof in such a manner as to correspond to the alignment groove 251m of the aspherical lens fixing portion 2511, and the alignment hole 251n communicates with the inner space of the body 251a. If it is desired to perform the alignment of the axis of the laser beam, accordingly, the fastening screw 251k inserted into the fourth through tap 251g4 of the cylindrical portion 251j through the alignment hole 251n from the outside is unfastened and fastened by a user's manipulation from the outside through the alignment hole 251n, thereby allowing the position of the center shaft of the cylindrical portion 251j to be adjusted. If it is desired to adjust the position of the center shaft of the aspherical lens fixing portion 2511 into which the aspherical lens 254 is mounted, moreover, the fastening screw 251k disposed on the cylindrical portion 251j is unfastened and a tool like a driver is inserted into the alignment groove 251m of the aspherical lens fixing portion 2511, thereby performing the adjustment of the position of the center shaft of the aspherical lens fixing portion 2511.

The hex bolts 251c, the headless bolts 251e, and the fastening bolts 251f are desirably made of aluminum materials having the same thermal expansion coefficient as each other.

The hex bolts 251c, the first through taps 251g1 and the non-through tap 251h, the headless bolts 251e and the second through taps 251g2, the fastening bolts 251f and the third through taps 251g3, and the fastening screw 251k and the alignment groove 251m serve as the laser beam alignment instruments for aligning the illumination direction of the laser beam. Since the light source portion 251 is sensitive to the vibrations caused by the external impacts when the vehicle on which the fluorescence detecting equipment is mounted is moved on rough places like the mountainous regions, as mentioned above, the laser beam alignment instruments are provided to perform the alignment of the axis of the laser beam.

That is, the laser beam generated from the laser diode 253 has a size of 1 x 2µm, and it is enlarged to a desired shape. However, if the illumination angle is a little changed by the external impacts, the laser beam is not located at the desired position, so that the illumination direction is aligned by using the above-mentioned alignment instruments to allow the laser beam to reach the particle measuring space S of the optical chamber 200.

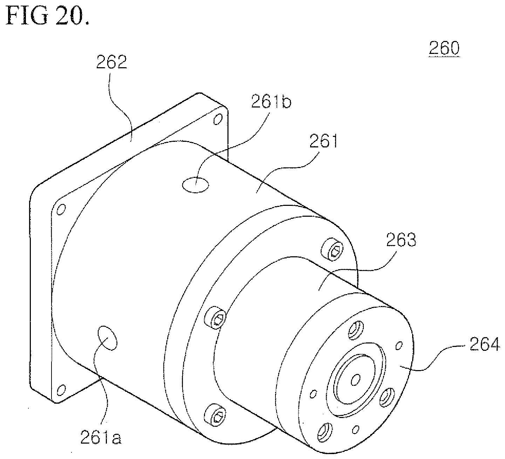

As shown in FIGS.20 to 23, the beam dump part 260 includes a body 261 fixed to the rear surface of the optical chamber 220 by means of screws in such a manner as to face the beam shaping part 250, a focal lens assembly 291 adapted to be inserted into an insertion through-hole 262a formed on the center of the body 261, a mounting recess 262b formed concavely on the front surface of the body 261 in such a manner as to have the insertion through-hole 262a positioned at the center thereof, a window 297 fixedly inserted into the mounting recess 262b, a fixing ring 290 fitted to the outer periphery of the focal lens assembly 291 in such a manner as to be covered by the window 297, a pin hole assembly 296 fitted to a concave portion formed on the rear surface of the body 261 in such a manner as to have the insertion through-hole 262a positioned at the center thereof, a housing 263 fixed to the rear end of the body 261 by means of screws, a light source output detector 264 fixed to the rear end of the housing 263 by means of screws, a sealing ring 298 fitted to a round groove formed on the center portion of the rear surface of the light source output detector 264, and a cover plate 299 fixed to the rear surface of the light source output detector 264.

The focal lens assembly 291 is adapted to allow the laser beam to be passed through a pin hole 293a (for example, a diameter of 0.3mm) of the pin hole assembly 296 by using a convex lens 292 as a focal lens.

The pin hole assembly 296 includes a cylindrical cap 293 having the pin hole 293a formed to be passed through the center of the front surface thereof, a plurality of blocks 294 (for example, four bocks) fitted between the outer periphery of the cylindrical cap 293 and the inner periphery of the concave portion formed on the rear surface of the body 261, and an end member 295 adapted to be brought into contact with the rear surface of the cylindrical cap 293.

The pin hole 293a is reduced in diameter toward the front surface of the cap 293.

The end member 295 has a through-hole formed on the center thereof in such a manner as to have a larger rear surface diameter than a front surface diameter thereof. In this case, the front surface diameter of the through-hole of the end member 295 is the same as the concave portion formed concavely on the rear surface of the cap 293.

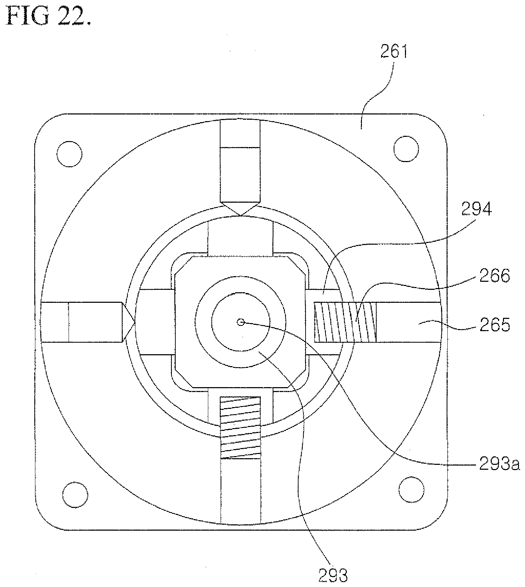

Next, the body 261 has a plurality of position adjusting members 265 (for example, four position adjusting members) spaced apart from each other along the outer periphery thereof in such a manner as to be penetratedly coupled thereto. As shown in FIG.22, the position adjusting members 265 have springs 266 adapted to pressurize the blocks 294 toward the outer periphery of the cap 293, so that the position of the cap 293 can be adjusted in the concave portion of the rear surface of the body 261 in accordance with the unfastening or fastening of the position adjusting members 265.

Further, the housing 263 has a hole formed on the center thereof, and the hole has a larger front surface diameter than a rear surface diameter thereof. In this case, the front surface diameter of the hole of the housing 263 is the same as the rear surface diameter of the through-hole formed on the center of the end member 295.

Also, the rear surface of the housing 263 is inclined by a given angle (for example, 10°) with respect to the reference surface perpendicular to an optical axis.

The light source output detector 264 detects the changes in the intensity of the laser beam if the beam shaping part 250 and the beam dump part 260 disposed at the front and back sides of the optical chamber 220 are not aligned to each other or if the beam shaping part 250 is abnormally operated, and generates warning through a warning device (not shown). A photodiode is used as the light source output detector 264.

Under the above-mentioned structure of the beam dump part 260, some of parts are fitted to each other and some of parts are integrated to each other by means of the fixation of screws, so that the assembling and disassembling works of the parts are performed easily and the mounting work on the device for real-time fluorescence detection is also carried out conveniently.

Furthermore, the opening formed between the front surface of the body 261 and the rear surface of the optical chamber 220 is sealed by means of the window 297 and the fixing ring 290, so that the internal pressure of the optical chamber 220 is maintained and the internal passage (or inner space) of the beam dump part 260 through which the laser beam is passed is cut off from the outside, thereby preventing air or light from the outside from entering the interior of the optical chamber 220 or the beam dump part 260. Accordingly, the accuracy and reliability of the measurement can be improved.

The laser beam (incident beam) generated from the beam shaping part 250 is illuminated to the fine particles toward the particle measuring space S of the optical chamber 220 and is passed through the window 297 of the beam dump part 260. After that, the laser beam is passed through the convex lens 292 of the focal lens assembly 291 and the pin hole 293a of the diameter of 0.3mm passed through the front surface of the cap 293 of the pin hole assembly 296 and then reaches the light source output detector 264. Further, the surface of the light source output detector 264 is inclined by an angle of 10° with respect to the reference surface perpendicular to the advancing passage of the incident beam (the optical axis). Under the above structure, even though a portion of the incident beam is reflected from the surface of the light source output detector 264 in the process of measuring the intensity of the laser beam, the reflected beam is generated in different passage from the incident beam, thereby substantially decreasing an amount of reflected beam going straight toward the pin hole 293a.

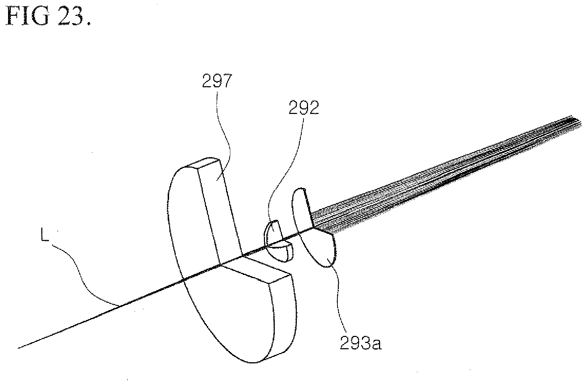

In more detail, as shown in FIG.23, the incident beam is collected to the pin hole 293a of the pin hole assembly 296 through the convex lens 292 of the focal lens assembly 291, and next, the incident beam passed through the pin hole 293a is passed through the cavity formed in the interior of the beam dump part 260 at the rear side of the pin hole 293a. Thus, as the incident beam comes near to the light source output detector 264, the width of the beam comes to be gradually expanded.

Like this, if the incident beam expanded in width reaches the light source output detector 264, the intensity of the incident beam is detected by the light source output detector 264 and a portion of the incident beam is reflected on the surface of the light source output detector 264.

The reflected beam generated from the surface of the light source output detector 264 is expanded in width as it comes near to the pin hole assembly 296 and is finally expanded in larger width by 20 times than the diameter of the pin hole 293a.

If it is assumed that the photodiode made of a silicone material is used as the light source output detector 264, the reflection rate when the incident beam is incident on the surface of the photodiode at a vertical angle (90°) is 30%, but the reflection rate when the incident beam is incident thereon with the inclination of a given angle (90°-10° = 80°) is just 0.075%. Referring to Gaussian distribution of beam, the energy of beam is just 0.2%.

According to the beam dump part 260 of the invention, most of reflected beam generated from the surface of the light source output detector 264 is not passed through the pin hole 293a. Further, even though the reflected beam is passed through the pin hole 293a, it is passed through the region where the outside intensity is low in Gaussian distribution, so that an extremely small amount of energy is introduced into the optical chamber 220.

As a result, the reflected light generated from the light source output detector 264 and the stray light generated in the beam dump part 260 are rarely introduced into the optical chamber 220, thereby improving the signal to noise ratio of the beam splitting part 270 and enhancing the accuracy and reliability of the measurement of the device for real-time fluorescence detection.

The beam splitting part 270 is connected to the optical chamber 220 in a horizontally supported state through a through-hole formed on a vertical support rod 203 fixed to the support plate 201 on the underside surface thereof.

The beam splitting part 270 is adapted to separate and detect weak scattering light and fluorescence collected through the spherical reflector 231 and the aspherical reflector 232 disposed in the particle measuring space S of the optical chamber 220. The beam splitting part 270 includes: a hollow housing 271 connected to the fixing plate 281 detachably coupled to the spherical reflector 231 in such a manner as to have the front surface located toward the spherical reflector 231 of the optical chamber 220; a first accommodating member 276 disposed inside the housing 271 to accommodate a first lens group 277a thereinto; a second accommodating member 278 disposed inside the housing 271 to accommodate two beam splitters 279a and 279b and second lens groups 277b and 277c thereinto; a photodiode (not shown) adapted to measure the output of the laser beam; a scattering light detector 274 (see FIG.6) disposed on the rear surface side of the housing 271 to separate scattering light in a determined direction in accordance with the cut-off frequencies of the beam splitters 279a and 279b; and two branched fluorescence detectors 272 and 273 connected vertically to the housing 271 by means of connection holes 271a and 271b formed on one side surface of the housing 271.

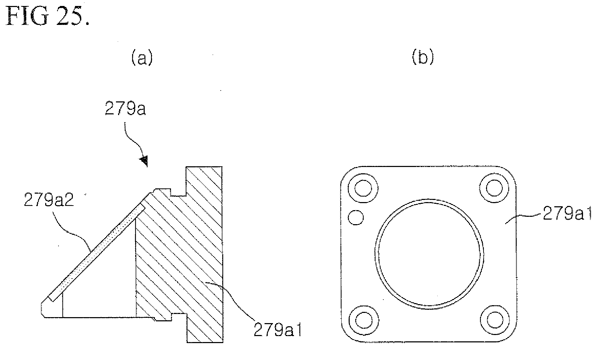

In this case, the beam splitters 279a and 279b have the same structure as each other, and as shown in FIGS.25a and 25b, each beam splitter includes: a body 279a1 having a mounting portion separably mounted on the housing 271 by means of fastening bolts through beam splitter mounting holes 271c and 271d formed spaced apart from the rear surface of the housing 271; and a reflecting member 279a2 disposed slantly on one surface of the body 279a1. Under the above structure, when the wavelength of the laser diode 253 is changed, the beam splitters 279a and 279b are easily exchanged.

One of the fluorescence detectors 272 and 273 is adapted to detect a long wavelength signal and the other to detect a short wavelength signal.

The scattering light detector 274 has an avalanche photodiode (APD) adapted to measure the scattering light signal generated from the particles and an amplifier. The gain of the avalanche photodiode is under the surrounding temperature, and accordingly, a sensor for measuring the surrounding temperature of the avalanche photodiode is desirably provided.

The fluorescence detectors 272 and 273 are desirably formed of photomultiplier tubes (PMT) for amplifying and detecting weak inherent fluorescence generated from the particles. An optical filter 275 is mounted on the front surface of each of the fluorescence detectors 272 and 273 to cut off the scattered light and pass the induced fluorescence therethrough.

The scattering light signal detected from the scattering light detector 274 is passed through a signal processing part (not shown) and is converted into particle sizes, and the fluorescence signals detected from the fluorescence detectors 272 and 273 are passed through the signal processing parts and are converted into fluorescence intensities.

As mentioned above, if the aerosol particles and a given amount of air are introduced into the optical chamber 220 through the inlet part 210 of the particle measuring unit 200, the scattering light is generated in the particle measuring space S formed in the optical chamber 220 by means of the fine particles passed through the laser beam illuminated from the beam shaping part 250 toward the beam dump part 260. The scattering light is detected by the scattering light detector 274, and especially, if the biological aerosols are contained in the particles, the fluorescence is also generated together with the scattering light. Accordingly, the fluorescence is detected by wavelength band to in real time monitor the size distribution and intensity changes of the fine particle in the air. Lastly, the detected scattering light and fluorescence are converted into electrical signals through a light detecting sensor and the state of the air is in real time analyzed by using a high speed signal processing circuit. Moreover, the intensity by particle size, the intensity of the fluorescent particles by particle size, and the intensity of the fluorescence by particle size of the fluorescence of the particles existing in the air are in real time analyzed in variable time unit, thereby detecting whether harmful materials exists or not.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A device for real-time fluorescence detection, which is adapted to separate and concentrate aerosol particles and to detect the fluorescence of the biological aerosols contained in the aerosol particles, the device comprising:
a particle concentrator (100) having a pre-separator (110) adapted to form a passage through which the aerosol particles are drawn into and to filter foreign materials and the particles of more than a given size from the aerosol particles, and a cylindrical casing (120) connected to the pre-separator (110) and having two-staged nozzles (121) and (122) formed of a plurality of virtual impactors adapted to separate and concentrate the particles passed through the pre-separator (110) to sizes within a given range through an inertial force; and
a particle measuring unit (200) located inside a casing assembling body (300) disposed on the underside of the particle concentrator (100),
**characterized in that** the particle measuring unit (200) comprises:
an inlet part (210) adapted to introduce the particles and air separated and concentrated in the particle concentrator (100) thereinto;
a rectangular parallelepiped optical chamber (220) connected to the lower end of the inlet part (210) through an opening (222) formed on the top surface thereof and having a particle measuring space S formed at the interior thereof;
a beam shaping part (250) connected to an opening (223) formed on the front surface of the optical chamber (220) to illuminate laser beam L to the particles introduced into the particle measuring space S through the inlet part (210);
a beam dump part (260) connected to an opening (225) formed on the rear surface of the optical chamber (220) in such a manner as to face the beam shaping part (250) to allow the laser beam L illuminated from the beam shaping part (250) to disappear;
a pair of reflectors (230) disposed in the particle measuring space S at an angle of 90° with respect to the forward direction of the laser beam L;
a particle discharging part (240) connected to an opening (227) formed on the bottom surface of the optical chamber (220) to discharge the particles and air interacting with the laser beam L in the particle measuring space S to the outside through a discharging nozzle (241);
a beam splitting part (270) connected to an opening (224) formed on the right surface of the optical chamber (220) in such a manner as to be perpendicular to the beam shaping part (250) and having a scattering light detector (274) and fluorescence detectors (272) and (273) adapted to detect one scattering light and two fluorescence at the same time in accordance with the cut-off frequencies of two beam splitters (279a) and (279b) from the scattering light and the fluorescence signal produced by the interaction between the laser beam L and the particles in the particle measuring space S of the optical chamber (220); and
the pair of reflectors (230) comprising a spherical reflector (231) made of a coated glass material and an aspherical reflector (232) made of a coated aluminum material, the spherical reflector (231) being disposed between the beam splitting part (270) and the aspherical reflector (232), and the spherical reflector (231) and the aspherical reflector (232) being disposed facing each other, the spherical reflector (231) being not coated on the centre portion thereof so that the scattering light and fluorescence signals generated by illuminating the laser beam L generated from the beam shaping part (250) on the particles introduced from the inlet part (210) are reflected to the aspherical reflector (232) and are advanced toward the beam splitting part (270).

2. The device for real-time fluorescence detection according to claim 1, wherein the pre-separator (110) of the particle concentrator (100) comprises: an absorption pipe (111) to which air and particles are drawn into; a filter (112) for removing foreign materials contained in the particles by using a mesh structure formed along the upper end periphery of the absorption pipe (111); a filter cap (113) adapted to cover the top end periphery of the filter (112) in such a manner as to allow the outer periphery thereof to be spaced apart from the filter (112); a funnel (114) disposed inside the absorption pipe (111) to filter particles having a larger size than the particles of a target size in the aerosol particles; and a cup (115) connected to the bottom end of the funnel (114) and having air passages (115a) formed on the side periphery thereof, into which the large particles filtered through the funnel (114) are put.

3. The device for real-time fluorescence detection according to claim 1, wherein the cylindrical casing (120) of the particle concentrator (100) comprises: the first stage nozzle (121) formed of a plurality of virtual impactors, through which the particles within a range of the given size in the particles passed through the air passages (115a) of the cup (115) are passed; and the second stage nozzle (122) spaced apart from the first stage nozzle (121) and formed of a plurality of virtual impactors, through which the particles within a range of the given size are separated and concentrated.

4. The device for real-time fluorescence detection according to claim 1, wherein the inlet part (210) comprises: a connection member (211) having an open portion penetrated in an up and down direction in such a manner as to be connected to the underside end of the particle concentrator (100); an outside nozzle (212) adapted to be separably coupled to the connection member (211) in such a manner as to be fixedly connected to the top surface opening (222) of the optical chamber (220) at the lower end portion thereof; an insertion tube (213) disposed at the interiors of the connection member (211) and the outside nozzle (212) and having an insertion hole into which the particle discharging port (123) protrudingly extended from the underside end of the second stage nozzle (122) is fitted; an inside nozzle (214) fixedly connected to the interior of the insertion tube (213); a nozzle tip (215) fixedly connected to the end of the outside nozzle (212); and an air introducing member (216) disposed on the outside of the outside nozzle (212) to introduce clean air from the outside thereinto.

5. The device for real-time fluorescence detection according to claim 4, wherein the inlet part (210) further comprises a first position determining means for determining the position of the inside nozzle (214) when the inside nozzle (214) is located in the connection member (211) and the outside nozzle (212), the first position determining means having a concave groove (213b) formed on one side of a ring member (213a) mounted along the outer periphery of the insertion tube (213) and a pin (212a) fixedly inserted into a pin hole formed on one side of the top periphery of the outside nozzle (212) in such a manner as to be inserted into the concave groove (213b).

6. The device for real-time fluorescence detection according to claim 4, wherein the inlet part (210) further comprises a second position determining means for determining the position of the inlet part (210) itself when the inlet part (210) is mounted on the optical chamber (220), the second position determining means having a concave groove (212d) formed on one side of a round projection (212c) formed on the outer periphery of the lower end portion of the outside nozzle (212) and a pin (221a) disposed on one side of the opening (222) formed on the top surface of the optical chamber (220) in such a manner as to be inserted into the concave groove (212d).

7. The device for real-time fluorescence detection according to claim 6, wherein the inlet part (210) is fixedly connected to the top surface opening (222) of the optical chamber (220) by means of screw-coupling between a nut member (217) having the upper inner periphery supported against the round projection (212c) formed on the outer periphery of the lower end portion of the outside nozzle (212a) and a screw thread formed on the inner periphery thereof and a connection member (221) protruded upwardly from the edge of the top surface opening (222) of the optical chamber (220) and having a screw thread formed on the outer periphery thereof.

8. The device for real-time fluorescence detection according to claim 7, wherein the pin (221a) is fixedly inserted into a pin hole formed on one side of the top surface periphery of the connection member (221).

9. The device for real-time fluorescence detection according to claim 4, wherein so as to allow the inlet part (210) to be aligned to the mounting position on the optical chamber (220), without any eccentricity in horizontal direction from the determined mounting position on the optical chamber (220) when the inlet part (210) is mounted again thereon after the separation from the optical chamber (220), the outside nozzle (212) of the inlet part (210) has an eccentricity prevention inclined portion (212e) formed on the lower end portion thereof, the eccentricity prevention inclined portion (212e) having a reversely conical structure in such a manner as to be inclined toward the top surface opening (222) of the optical chamber (220), and the top surface opening (222) of the optical chamber (220) has an inclined surface (222a) formed on the lower end of the inner periphery thereof in such a manner as to be brought into close contact with the eccentricity prevention inclined portion (212e).

10. The device for real-time fluorescence detection according to claim 9, wherein the inclined angles of the eccentricity prevention inclined portion (212e) and the inclined surface (222a) are the same as each other, and they are in a range between 25° and 35° with respect to the center axis of the inlet part (210).

11. The device for real-time fluorescence detection according to claim 1, wherein the discharging nozzle (241) of the particle discharging part (240) has a tapered structure being reduced in diameter toward the front end portion thereof, while being disposed to face the nozzle tip (215) connected to the end of the outside nozzle (212) of the inlet part (210) in a state of being spaced apart by a given distance from the nozzle tip (215).

12. The device for real-time fluorescence detection according to claim 1, wherein the beam shaping part (250) comprises: a light source portion (251) having a body (251a) having an inner space open at both end portions thereof to mount an aspherical lens (254) thereinto and a cover plate (251b) having a socket in which a laser diode (253) is embedded at the front of the body (251a); and a beam forming and adjusting portion (252) separably coupled to the light source portion (251) and having a first lens group (255), a second lens group (256) and a window (257) sequentially arranged thereinto, the first lens group (255) adapted to adjust the size in the longitudinal direction of the laser beam generated from the laser diode (253) and passed through the aspherical lens (254), and the second lens group (256) adapted to adjust the size in the transverse direction of the laser beam passed through the first lens group (255), and the beam shaping part (250) is blackened on the inner surface thereof.

13. The device for real-time fluorescence detection according to claim 12, wherein the light source portion (251) of the beam shaping part (250) comprises a laser beam alignment means having a plurality of hex bolts (251c) inserted through a plurality of first through taps (251g1) formed spaced apart from each other on the inner edge periphery of the cover plate (251b) and a plurality of non-through taps (251h) formed on the body (251a) in such a manner as to correspond to the first through taps (251g1), each hex bolt (251c) having a spring (251d) mounted on the outer periphery thereof to adjust the pressurizing force of the cover plate (251b) toward the body (251a), and a plurality of headless bolts (251e) inserted through a plurality of second through taps (251g2) formed between the neighboring first through taps (251g1) formed on the cover plate (251b) to adjust the pushing force of the cover plate (251b) from the body (251a) in accordance with the fastening degree thereof.

14. The device for real-time fluorescence detection according to claim 13, wherein each headless bolt (251e) is inserted into a bushing (251i) fitted to the second through tap (251g2) and has a ball inserted into the end thereof to perform torque offset, the bushing (251i) having an incision groove (251i1) formed on one side surface in a longitudinal direction thereof.

15. The device for real-time fluorescence detection according to claim 13, wherein the laser beam alignment means further comprises fastening bolts (251f1, 251f2 and 251f3) inserted through three groups of third through taps (251g3) spaced apart from each other along the periphery of the cover plate (251a) in such a manner as to communicate with the second through taps (251g2), to fasten and fix the bushings (251i) into which the headless bolts (251e) are inserted.

16. The device for real-time fluorescence detection according to claim 15, wherein each group of third through taps (251g3) is formed of three through taps of a triangular shape.

17. The device for real-time fluorescence detection according to claim 13, wherein the light source portion (251) of the beam shaping part (250) further comprises: a cylindrical portion (251j) formed on one side surface of the cover plate (251b) in such a manner as to be protruded horizontally toward the body (251a) and having an inner space through which the laser beam L generated from the laser diode (253) is passed; a fixing plate (251o) disposed at the inside of the front end portion of the body (251a) in such a manner as to have an open portion formed to accommodate the cylindrical portion (251j) thereinto in the state of surrounding the outer periphery of the cylindrical portion (251j), the fixing plate (251o) being fixed to the body (251a) by means of a fastening bolt (251p); and an aspherical lens fixing portion (2511) mounted on the inner space of the cylindrical portion (251j) and having open both end portions thereof in such a manner as to fix the aspherical lens (254) to the inner space thereof.

18. The device for real-time fluorescence detection according to claim 17, wherein the cylindrical portion (251j) has a fourth through tap (251g4) formed on the top surface thereof in such a manner as to communicate with the inner space thereof, the fourth through tap (251g4) having a fastening screw (251k) inserted thereinto, the aspherical lens fixing portion (2511) has an alignment groove (251m) formed on the top surface of the rear end portion thereof to align the position of the aspherical lens fixing portion (2511), and the body (251a) of the light source portion (251) has an alignment hole (251n) formed on one side of the top surface thereof in such a manner as to communicate with the inner space of the body (251a) and to correspond to the fastening screw (251k) inserted into the fourth through tap (251g4) of the cylindrical portion (251j) and the position of the alignment groove (251m) of the aspherical lens fixing portion (2511), so as to allow the position of the center shaft of the cylindrical portion (251j) and the position of the center shaft of the aspherical lens fixing portion (2511) to be adjusted from the outside.

19. The device for real-time fluorescence detection according to claim 1, wherein the beam dump part (260) comprises: a body (261) fixed to the rear surface of the optical chamber (220); a focal lens assembly (291) adapted to be inserted into an insertion through-hole (262a) formed on the center of the body 261; a pin hole assembly (296) fitted to a concave portion formed on the rear surface of the body (261) in such a manner as to allow the optical axis of the focal lens assembly (291) to be passed through a pin hole (293a); a housing (263) fixed to the rear end of the pin hole assembly (296); and a light source output detector (264) fixed to the rear end of the housing (263) in such a manner as to be inclined by a given angle with respect to the reference surface perpendicular to the optical axis of the focal lens assembly (291), the pin hole assembly (296) and the housing (264) having a cavity formed on the center portions thereof to allow the laser beam L passed through the pin hole (293a) of the pin hole assembly (296) to be expanded in width as it comes near to the light source output detector (264).

20. The device for real-time fluorescence detection according to claim 1, wherein the aspherical reflector (232) is disposed on the opening (226) formed on the left side surface of the optical chamber (220), and the opening (226) is closed by a separable sealing plate (280).

21. The device for real-time fluorescence detection according to claim 1, wherein the scattering light detector (274) has an avalanche photodiode (APD) and an amplifier.

22. The device for real-time fluorescence detection according to claim 1, wherein one of the fluorescence detectors (272) and (273) is adapted to detect a long wavelength signal and the other to detect a short wavelength signal, each fluorescence detector having an optical filter (275) mounted on the front surface thereof to cut off the scattered light and pass the induced fluorescence therethrough.

23. The device for real-time fluorescence detection according to claim 12, wherein the fluorescence detectors (272) and (273) are formed of photomultiplier tubes (PMTs).

## Patentansprüche

1. Vorrichtung zur Echtzeitfluoreszenzdetektion, die dazu ausgelegt ist, Aerosolpartikel zu separieren und zu konzentrieren, und die Fluoreszenz der biologischen Aerosole zu detektieren, die in den Aerosolpartikeln enthalten sind, wobei die Vorrichtung umfasst:
einen Partikelkonzentrator (100) mit einem Vorseparator (110), der dazu ausgelegt ist, einen Durchgang zu bilden, durch den die Aerosolpartikel eingezogen werden, und Fremdmaterialien sowie die Partikel von mehr als einer vorgegebenen Größe von den Aerosolpartikeln auszufiltern, und ein zylindrisches Gehäuse (120), das mit dem Vorseparator (110) verbunden ist und zweistufige Düsen (121) und (122) hat, die aus einer Mehrzahl von virtuellen Stoßkörpern gebildet sind, die dazu ausgelegt sind, die durch den Vorseparator (110) hindurchgegangenen Partikel auf Größen innerhalb eines gegebenen Bereichs durch eine Trägheitskraft zu separieren und zu konzentrieren; und
eine Partikelmesseinheit (200), die innerhalb eines Gehäusemontagekörpers (300) lokalisiert ist, der an der Unterseite des Partikelkonzentrators (100) angeordnet ist,
**dadurch gekennzeichnet, dass** die Partikelmesseinheit (200) umfasst:
ein Einlassteil (210), das dazu ausgelegt ist, die Partikel und Luft, die in dem Partikelkonzentrator (100) separiert und konzentriert sind, darin einzubringen;
eine quaderförmige optische Kammer (220), die mit dem unteren Ende des Einlassteils (210) durch eine Öffnung (222) verbunden ist, die an der oberen Oberfläche davon gebildet ist, und einen Partikelmessraum S hat, der in ihrem Inneren gebildet ist;
ein Strahlformungsteil (250), das mit einer Öffnung (223) verbunden ist, die an der vorderen Oberfläche der optischen Kammer (220) gebildet ist, um einen Laserstrahl L auf die Partikel zu strahlen, die durch das Einlassteil (210) in den Partikelmessraum S eingebracht sind;
ein Strahlabfangteil (260), das mit einer Öffnung (225) verbunden ist, die an der hinteren Oberfläche der optischen Kammer (220) gebildet ist, derart, dass es dem Strahlformungsteil (250) gegenüber liegt, um es dem von dem Strahlformungsteil (250) eingestrahlten Laserstrahl L zu ermöglichen, zu verschwinden;
ein Paar von Reflektoren (230), die in dem Partikelmessraum S unter einem Winkel von 90° mit Bezug zur Vorwärtsrichtung des Laserstrahls L angeordnet sind;
ein Partikelabgabeteil (240), das mit einer Öffnung (227) verbunden ist, die an der Bodenoberfläche der optischen Kammer (220) gebildet ist, um die Partikel und Luft, die mit dem Laserstrahl L in dem Partikelmessraum S interagieren, durch eine Abgabedüse (241) nach außen abzugeben;
ein Strahlteilerteil (270), das mit einer Öffnung (224) verbunden ist, die an der rechten Oberfläche der optischen Kammer (220) gebildet ist, derart, dass es orthogonal zu dem Strahlformungsteil (250) ist, und das einen Streulichtdetektor (274) und Fluoreszenzdetektoren (272) und (273) hat, die dazu ausgelegt sind, erstens Streulicht und zweitens Fluoreszenz gleichzeitig in Übereinstimmung mit den Abschneidefrequenzen von zwei Strahlteilern (279a) und (279b) von dem Streulicht und dem Fluoreszenzsignal zu detektieren, das durch die Interaktion zwischen dem Laserstrahl L und den Partikeln in dem Partikelmessraum S der optischen Kammer (220) erzeugt ist; und
wobei das Paar von Reflektoren (230) einen sphärischen Reflektor (231) umfasst, der aus einem beschichteten Glasmaterial gefertigt ist, und einen asphärischen Reflektor (232), der aus einem beschichteten Aluminiummaterial gefertigt ist, wobei der sphärische Reflektor (231) zwischen dem Strahlteilerteil (270) und dem asphärischen Reflektor (232) angeordnet ist, und wobei der sphärische Reflektor (231) und der asphärische Reflektor (232) einander gegenüberliegend angeordnet sind, wobei der sphärische Reflektor (231) an seinem Zentralbereich nicht beschichtet ist, so dass das Streulicht und Fluoreszenzsignale, die erzeugt sind durch Einstrahlen des Laserstrahls L, erzeugt von dem Strahlformungsteil (250), auf die von dem Einlassteil (210) eingebrachten Partikel zu dem asphärischen Reflektor (232) reflektiert und zu dem Strahlteilerteil (270) weitergeleitet werden.

2. Vorrichtung zur Echtzeitfluoreszenzdetektion nach Anspruch 1, wobei der Vorseparator (110) des Partikelkonzentrators (100) umfasst: ein Absorptionsrohr (111), in das Luft und Partikel eingezogen werden; einen Filter (112) zum Entfernen von Fremdmaterialien, die in den Partikeln enthalten sind, durch Verwendung einer Maschenstruktur, die entlang des oberen Endumfangs des Absorptionsrohrs (111) gebildet ist; eine Filterkappe (113), die dazu ausgelegt ist, den oberen Endumfang des Filters (112) derart zu bedecken, dass sein Außenumfang von dem Filter (112) beabstandet werden kann; einen Trichter (114), der innerhalb des Absorptionsrohrs (111) angeordnet ist, um Partikel auszufiltern, die eine größere Größe als die Partikel einer Sollgröße in den Aerosolpartikeln haben; und einen Becher (115), der mit dem Bodenende des Trichters (114) verbunden ist, und Luftdurchgänge (115a) hat, die an seinem Seitenumfang gebildet sind, in den die durch den Trichter (114) ausgefilterten großen Partikel gelangen.

3. Vorrichtung zur Echtzeitfluoreszenzdetektion nach Anspruch 1, wobei das zylindrische Gehäuse (120) des Partikelkonzentrators (100) umfasst: die Erststufendüse (121), die aus einer Mehrzahl von virtuellen Stoßkörpern gebildet ist, durch die die Partikel innerhalb eines Bereichs der gegebenen Größe in den Partikeln, die durch die Luftdurchgänge (115a) des Bechers (115) hindurchgegangen sind, hindurchgehen; und die Zweitstufendüse (122), die von der Erststufendüse (121) beabstandet ist und aus einer Mehrzahl von virtuellen Stoßkörpern gebildet ist, durch die die Partikel innerhalb eines Bereichs der gegebenen Größe separiert und konzentriert werden.

4. Vorrichtung zur Echtzeitfluoreszenzdetektion nach Anspruch 1, wobei das Einlassteil (210) umfasst: ein Verbindungselement (211) mit einem offenen Bereich, der in einer Auf-Ab-Richtung derart durchdrungen ist, dass es mit dem Unterseitenende des Partikelkonzentrators (100) verbunden ist; eine Außendüse (212), die dazu ausgelegt ist, trennbar mit dem Verbindungselement (211) derart gekoppelt zu sein, dass sie fest mit der oberen Oberflächenöffnung (222) der optischen Kammer (220) an dem unteren Endbereich davon verbunden ist; ein Einsetzrohr (213), das im Inneren des Verbindungselements (211) und der Außendüse (212) angeordnet ist und ein Einsetzloch hat, in das der Partikelabgabeanschluss (123) eingepasst ist, der sich vorstehend von dem Unterseitenende der Zweitstufendüse (122) erstreckt; einen Innendüse (214), die fest mit dem Inneren des Einsetzrohrs (213) verbunden ist; eine Düsenspitze (215), die fest mit dem Ende der Außendüse (212) verbunden ist; und ein Lufteinbringelement (216), das an der Außenseite der Außendüse (212) angeordnet ist, um Frischluft von der Außenseite darin einzubringen.

5. Vorrichtung zur Echtzeitfluoreszenzdetektion nach Anspruch 4, wobei das Einlassteil (210) ferner eine erste Positionsbestimmungseinrichtung umfasst zum Bestimmen der Position der Innendüse (214), wenn die Innendüse (214) in dem Verbindungselement (211) und der Außendüse (212) lokalisiert ist, wobei die erste Positionsbestimmungseinrichtung eine konkave Rille (213b) hat, die an einer Seite eines Ringelements (213a) gebildet ist, das entlang des Außenumfangs des Einsetzrohrs (213) montiert ist, und einen Stift (212a), der fest in ein Stiftloch eingesetzt ist, das an einer Seite des oberen Umfangs der Außendüse (212) gebildet ist, derart, dass er in die konkave Rille (213b) eingesetzt ist.

6. Vorrichtung zur Echtzeitfluoreszenzdetektion nach Anspruch 4, wobei das Einlassteil (210) ferner eine zweite Positionsbestimmungseinrichtung umfasst zur Bestimmung der Position des Einlassteils (210) selbst, wenn das Einlassteil (210) an der optischen Kammer (220) montiert ist, wobei die zweite Positionsbestimmungseinrichtung eine konkave Rille (212d) hat, die an einer Seite eines runden Vorsprungs (212c) gebildet ist, der am Außenumfang des unteren Endbereichs der Außendüse (212) gebildet ist, und einen Stift (221a), der an einer Seite der Öffnung (222) angeordnet ist, die an der oberen Oberfläche der optischen Kammer (220) gebildet ist, derart, dass er in die konkave Rille (212d) eingesetzt ist.

7. Vorrichtung zur Echtzeitfluoreszenzdetektion nach Anspruch 6, wobei das Einlassteil (210) fest mit der oberen Oberflächenöffnung (222) der optischen Kammer (220) verbunden ist mittels einer Schraubverbindung zwischen einem Mutterelement (217), dessen oberer Innenumfang gegen den runden Vorsprung (212c) abgestützt ist, der am Außenumfang des unteren Endbereichs der Außendüse (212a) gebildet ist, und einem Schraubgewinde, das am Innenumfang davon gebildet ist, und einem Verbindungselement (221), das aufwärts vom Rand der oberen Oberflächenöffnung (222) der optischen Kammer (220) vorsteht und ein Schraubengewinde hat, das am Außenumfang davon gebildet ist.

8. Vorrichtung zur Echtzeitfluoreszenzdetektion nach Anspruch 7, wobei der Stift (221a) fest in ein Stiftloch eingesetzt ist, das an einer Seite des oberen Oberflächenumfangs des Verbindungselements (221) gebildet ist.

9. Vorrichtung zur Echtzeitfluoreszenzdetektion nach Anspruch 4, wobei zum Ermöglichen, dass das Einlassteil (210) mit der Montageposition an der optischen Kammer (220) ausgerichtet wird ohne Exzentrizität in horizontaler Richtung von der bestimmten Montageposition an der optischen Kammer (220), wenn das Einlassteil (210) nach der Trennung von der optischen Kammer (220) wieder daran montiert wird, die Außendüse (212) des Einlassteils (210) einen geneigten Exzentrizitätsverhinderungsbereich (212e) hat, der am unteren Endbereich davon gebildet ist, wobei der geneigte Exzentrizitätsverhinderungsbereich (212e) eine umgekehrte konische Struktur derart hat, dass er zur oberen Oberflächenöffnung (222) der optischen Kammer (220) geneigt ist, und wobei die obere Oberflächenöffnung (222) der optischen Kammer (220) eine geneigte Oberfläche (222a) hat, die am unteren Ende des Innenumfangs davon gebildet ist, derart, dass sie in engen Kontakt mit dem geneigten Exzentrizitätsverhinderungsbereich (212e) gebracht wird.

10. Vorrichtung zur Echtzeitfluoreszenzdetektion nach Anspruch 9, wobei die Neigungswinkel des geneigten Exzentrizitätsverhinderungsbereichs (212e) und der geneigten Oberfläche (222a) zueinander gleich sind, und sie in einem Bereich zwischen 25° und 35° mit Bezug zur Mittelachse des Einlassteils (210) sind.

11. Vorrichtung zur Echtzeitfluoreszenzdetektion nach Anspruch 1, wobei die Abgabedüse (241) des Partikelabgabeteils (240) eine sich verjüngende Struktur hat, deren Durchmesser in Richtung des vorderen Endbereichs davon geringer wird, während sie so angeordnet ist, dass sie der Düsenspitze (215) gegenüber liegt, die mit dem Ende der Außendüse (212) des Einlassteils (210) verbunden ist, in einem Zustand der Trennung von der Düsenspitze (215) um einen gegebenen Abstand.

12. Vorrichtung zur Echtzeitfluoreszenzdetektion nach Anspruch 1, wobei das Strahlformungsteil (250) umfasst: einen Lichtquellenbereich (251) mit einem Körper (251a), der einen Innenraum hat, der an beiden Endbereichen davon offen ist, um eine asphärische Linse (254) darin zu montieren, und eine Abdeckplatte (251b) mit einer Fassung, in der eine Laserdiode (253) an der Vorderseite des Körpers (251a) eingebettet ist; und einen Strahlformungs- und Einstellbereich (252), der trennbar mit dem Lichtquellenbereich (251) gekoppelt ist und eine erste Linsengruppe (255), eine zweite Linsengruppe (256) und ein Fenster (257) hat, die sequentiell darin angeordnet sind, wobei die erste Linsengruppe (255) dazu ausgelegt ist, die Größe des Laserstrahls in der Längsrichtung einzustellen, der von der Laserdiode (253) erzeugt ist und durch die asphärische Linse (254) hindurch gegangen ist, und wobei die zweite Linsengruppe (256) dazu ausgelegt ist, die Größe des Laserstrahls in der Querrichtung einzustellen, der durch die erste Linsengruppe (255) hindurch gegangen ist, und wobei das Strahlformungsteil (250) an seiner Innenoberfläche geschwärzt ist.

13. Vorrichtung zur Echtzeitfluoreszenzdetektion nach Anspruch 12, wobei der Lichtquellenbereich (251) des Strahlformungsteils (250) eine Laserstrahlausrichteinrichtung umfasst, die eine Mehrzahl von Sechskantbolzen (251c) hat, die durch eine Mehrzahl von ersten Durchgangsbohrungen (251g1) eingesetzt sind, die voneinander beabstandet am Innenrandumfang der Abdeckplatte (251b) gebildet sind, und eine Mehrzahl von Nichtdurchgangsbohrungen (251h), die an dem Körper (251a) derart gebildet sind, dass sie den ersten Durchgangsbohrungen (251g1) entsprechen, wobei jeder Sechskantbolzen (251c) eine Feder (251d) hat, die am Außenumfang davon montiert ist, um die Drückkraft der Abdeckplatte (251b) in Richtung des Körpers (251a) einzustellen, und eine Mehrzahl von Madenbolzen (251e), die durch eine Mehrzahl von zweiten Durchgangsbohrungen (251 g2) eingesetzt sind, die zwischen den benachbarten ersten Durchgangsbohrungen (251g1) gebildet sind, die an der Abdeckplatte (251b) gebildet sind, um die Drückkraft der Abdeckplatte (251b) von dem Körper (251a) in Übereinstimmung mit dem Befestigungsgrad davon einzustellen.

14. Vorrichtung zur Echtzeitfluoreszenzdetektion nach Anspruch 13, wobei jeder Madenbolzen (251e) in eine Buchse (251i) eingesetzt ist, die in die zweite Durchgangsbohrung (251g2) eingepasst ist, und einen Ball hat, der in das Ende davon eingesetzt ist, um einen Drehmomentausgleich durchzuführen, wobei die Buchse (251i) eine Einschnittrille (251i1) hat, die an einer Seitenoberfläche in einer Längsrichtung davon gebildet ist.

15. Vorrichtung zur Echtzeitfluoreszenzdetektion nach Anspruch 13, wobei die Laserstrahlausrichteinrichtung ferner Befestigungsbolzen (251f1, 251f2 und 251f3) umfasst, die durch drei Gruppen von dritten Durchgangsbohrungen (251g3) eingesetzt sind, die voneinander entlang dem Umfang der Abdeckplatte (251a) beabstandet sind, derart, dass sie mit den zweiten Durchgangsbohrungen (251g2) kommunizieren, um die Buchsen (251i) zu befestigen und fixieren, in die die Madenbolzen (251e) eingesetzt sind.

16. Vorrichtung zur Echtzeitfluoreszenzdetektion nach Anspruch 15, wobei jede Gruppe von dritten Durchgangsbohrungen (251g3) aus drei Durchgangsbohrungen mit einer dreieckigen Gestalt gebildet ist.

17. Vorrichtung zur Echtzeitfluoreszenzdetektion nach Anspruch 13, wobei der Lichtquellenbereich (251) des Strahlformungsteils (250) ferner umfasst: einen zylindrischen Bereich (251j), der an einer Seitenoberfläche der Abdeckplatte (251b) derart gebildet ist, dass er horizontal zu dem Körper (251a) vorsteht und einen Innenraum hat, durch den der Laserstrahl L hindurch geht, der von der Laserdiode (253) erzeugt wird; eine Befestigungsplatte (251o), die an der Innenseite des vorderen Endbereichs des Körpers (251a) derart angeordnet ist, dass sie einen offenen Bereich hat, der dazu gebildet ist, den zylindrischen Bereich (251j) darin in dem Zustand des Umgebens des Außenumfangs des zylindrischen Bereichs (251j) aufzunehmen, wobei die Befestigungsplatte (251o) an dem Körper (251a) mittels eines Befestigungsbolzens (251p) befestigt ist; und einen Asphärische-Linse-Befestigungsbereich (2511), der am Innenraum des zylindrischen Bereichs (251j) montiert ist und offene beidendige Bereiche davon hat, derart, dass die asphärische Linse (254) an dem Innenraum davon befestigt ist.

18. Vorrichtung zur Echtzeitfluoreszenzdetektion nach Anspruch 17, wobei der zylindrische Bereich (251j) eine vierte Durchgangsbohrung (251g4) hat, die an der oberen Oberfläche davon derart gebildet ist, dass sie mit dem Innenraum davon kommuniziert, wobei die vierte Durchgangsbohrung (251g4) eine darin eingesetzte Befestigungsschraube (251k) hat, wobei der Asphärische-Linse-Befestigungsbereich (2511) eine Ausrichtrille (251m) hat, die an der oberen Oberfläche des hinteren Endbereichs davon gebildet ist, um die Position des Asphärische-Linse-Befestigungsbereichs (2511) auszurichten, und wobei der Körper (251a) des Lichtquellenbereichs (251) ein Ausrichtloch (251n) hat, das an einer Seite der oberen Oberfläche davon derart gebildet ist, dass es mit dem Innenraum des Körpers (251a) kommuniziert und der Befestigungsschraube (251k) entspricht, die in die vierte Durchgangsbohrung (251g4) des zylindrischen Bereichs (251j) eingesetzt ist, und der Position der Ausrichtrille (251m) des Asphärische-Linse-Befestigungsbereichs (2511), um zu ermöglichen, dass die Position der Zentralwelle des zylindrischen Bereichs (251j) und die Position der Zentralwelle des Asphärische-Linse-Befestigungsbereichs (2511) von außen eingestellt werden.

19. Vorrichtung zur Echtzeitfluoreszenzdetektion nach Anspruch 1, wobei das Strahlabfangteil (260) umfasst: einen Körper (261), der an der hinteren Oberfläche der optischen Kammer (220) befestigt ist; eine Fokallinsenanordnung (291), die dazu ausgelegt ist, in ein Einsetzdurchgangsloch (262a) eingesetzt zu werden, das in der Mitte des Körpers (261) gebildet ist; eine Pinholeanordnung (296), die in einem konkaven Bereich eingepasst ist, der an der hinteren Oberfläche des Körpers (261) gebildet ist, derart, dass die optische Achse der Fokallinsenanordnung (291) durch ein Pinloch (293a) hindurch geht; ein Gehäuse (263), das an dem hinteren Ende der Pinholeanordnung (296) befestigt ist; und einen Lichtquellenausgabedetektor (264), der an dem hinteren Ende des Gehäuses (263) derart befestigt ist, dass er um einen gegebenen Winkel mit Bezug zur Referenzoberfläche orthogonal zur optischen Achse der Fokallinsenanordnung (291) geneigt ist, wobei die Pinholeanordnung (296) und das Gehäuse (264) einen Hohlraum haben, der an den Zentralbereichen davon gebildet ist, um zu ermöglichen, dass der Laserstrahl L, der durch das Pinhole (293a) der Pinholeanordnung (296) hindurch gegangen ist, in der Breite aufgeweitet wird, wenn er sich dem Lichtquellenausgabedetektor (264) nähert.

20. Vorrichtung zur Echtzeitfluoreszenzdetektion nach Anspruch 1, wobei der asphärische Reflektor (232) an der Öffnung (226) angeordnet ist, die an der linken Seitenoberfläche der optischen Kammer (220) gebildet ist, und wobei die Öffnung (226) durch eine abtrennbare Abdichtplatte (280) verschlossen ist.

21. Vorrichtung zur Echtzeitfluoreszenzdetektion nach Anspruch 1, wobei der Streulichtdetektor (274) eine Avalanche-Fotodiode (APD) und einen Verstärker hat.

22. Vorrichtung zur Echtzeitfluoreszenzdetektion nach Anspruch 1, wobei einer der Fluoreszenzdetektoren (272) und (273) dazu ausgelegt ist, ein Lange-Wellenlänge-Signal zu detektieren, und der andere dazu ausgelegt ist, ein Kurze-Wellenlänge-Signal zu detektieren, wobei jeder Fluoreszenzdetektor einen optischen Filter (275) hat, der an der vorderen Oberfläche davon montiert ist, um das Streulicht abzuschneiden und die induzierte Fluoreszenz hindurch gehen zu lassen.

23. Vorrichtung zur Echtzeitfluoreszenzdetektion nach Anspruch 12, wobei die Fluoreszenzdetektoren (272) und (273) aus Photomultiplier-Röhren (PMTs) gebildet sind.

## Revendications

1. Dispositif de détection de fluorescence en temps réel, qui est adapté pour séparer et concentrer des particules d'aérosol et pour détecter la fluorescence des aérosols biologiques contenus dans les particules d'aérosol, le dispositif comprenant :
un concentrateur de particules (100) qui possède un pré-séparateur (110) adapté pour former un passage par lequel les particules d'aérosol sont aspirées et pour filtrer les corps étrangers et les particules supérieures à une taille donnée parmi les particules d'aérosol, et un boîtier cylindrique (120) relié au pré-séparateur (110) et qui possède des buses à deux étages (121) et (122) composées d'une pluralité d'impacteurs virtuels adaptés pour séparer et concentrer les particules qui sont passées par le pré-séparateur (110) selon des tailles données par le biais d'une force d'inertie ; et
une unité de mesure de particules (200) située à l'intérieur d'un corps d'assemblage de boîtier (300) disposé sur la face inférieure du concentrateur de particules (100),
**caractérisé en ce que** l'unité de mesure de particules (200) comprend :
une partie d'admission (210) adaptée pour introduire les particules et l'air séparés et concentrés dans le concentrateur de particules (100) à l'intérieur ;
une chambre optique parallélépipède rectangle (220) reliée à l'extrémité inférieure de la partie d'admission (210) par le biais d'une ouverture (222) formée sur la surface supérieure de celle-ci et qui possède un espace de mesure de particules S formé au niveau de son intérieur ;
une partie de mise en forme de faisceau (250) reliée à une ouverture (223) formée sur la surface avant de la chambre optique (220) afin d'illuminer un faisceau laser L vers les particules introduites dans l'espace de mesure de particules S par le biais de la partie d'admission (210) ;
une partie de décharge de faisceau (260) reliée à une ouverture (225) formée sur la surface arrière de la chambre optique (220) de façon à faire face à la partie de mise en forme de faisceau (250) afin de permettre au faisceau laser L illuminé par la partie de mise en forme de faisceau (250) de disparaître ;
une paire de réflecteurs (230) disposée dans l'espace de mesure de particules S à un angle de 90° par rapport à la direction avant du faisceau laser L ;
une partie d'évacuation de particules (240) reliée à une ouverture (227) formée sur la surface inférieure de la chambre optique (220) afin d'évacuer les particules et l'air qui interagissent avec le faisceau laser L dans l'espace de mesure de particules S vers l'extérieur par le biais d'une buse d'évacuation (241) ;
une partie de séparation de faisceau (270) reliée à une ouverture (224) formée sur la surface de droite de la chambre optique (220) de façon à être perpendiculaire à la partie de mise en forme de faisceau (250), et qui possède un détecteur de lumière de diffusion (274) et des détecteurs de fluorescence (272) et (273) adaptés pour détecter une lumière de diffusion et deux fluorescences en même temps selon les fréquences de coupure de deux séparateurs de faisceau (279a) et (279b) à partir de la lumière de diffusion et du signal de fluorescence produit par l'interaction entre le faisceau laser L et les particules dans l'espace de mesure de particules S de la chambre optique (220) ;
et
la paire de réflecteurs (230) comprenant un réflecteur sphérique (231) composé d'un matériau en verre enduit et un réflecteur asphérique (232) composé d'un matériau en aluminium enduit, le réflecteur sphérique (231) étant disposé entre la partie de séparation de faisceau (270) et le réflecteur asphérique (232), et le réflecteur sphérique (231) et le réflecteur asphérique (232) étant disposés face à face, le réflecteur sphérique (231) n'étant pas enduit sur sa partie centrale de sorte que la lumière de diffusion et les signaux de fluorescence générés en illuminant le faisceau laser L généré par la partie de mise en forme de faisceau (250) sur les particules introduites depuis la partie d'admission (210) soient réfléchis vers le réflecteur asphérique (232) et soient avancés vers la partie de séparation de faisceau (270).

2. Dispositif de détection de fluorescence en temps réel selon la revendication 1, dans lequel le pré-séparateur (110) du concentrateur de particules (100) comprend : un tuyau d'absorption (111) vers lequel de l'air et des particules sont aspirés ; un filtre (112) destiné à éliminer les corps étrangers contenus dans les particules à l'aide d'une structure à maillage formée le long de la périphérie d'extrémité supérieure du tuyau d'absorption (111) ; un embout de filtre (113) adapté pour recouvrir la périphérie d'extrémité supérieure du filtre (112) de façon à permettre à sa périphérique extérieure d'être espacée du filtre (112) ; un entonnoir (114) disposé à l'intérieur du tuyau d'absorption (111) afin de filtrer les particules qui présentent une taille supérieure aux particules d'une taille cible parmi les particules d'aérosol ; et une coupelle (115) reliée à l'extrémité inférieure de l'entonnoir (114) et qui possède des passages d'air (115a) formés sur sa périphérie latérale, dans laquelle les particules de grande taille filtrées par l'entonnoir (114) sont placées.

3. Dispositif de détection de fluorescence en temps réel selon la revendication 1, dans lequel le boîtier cylindrique (120) du concentrateur de particules (100) comprend : la buse de premier étage (121) formée d'une pluralité d'impacteurs virtuels, par laquelle passent les particules d'une taille donnée parmi les particules qui sont passées par les passages d'air (115a) de la coupelle (115) ; et la buse de second étage (122) espacée de la buse de premier étage (121) et composée d'une pluralité d'impacteurs virtuels, par laquelle les particules d'une taille donnée sont séparées et concentrées.

4. Dispositif de détection de fluorescence en temps réel selon la revendication 1, dans lequel la partie d'admission (210) comprend : un élément de liaison (211) qui possède une partie ouverte pénétrée vers le haut et vers le bas de façon à être reliée à l'extrémité de la face inférieure du concentrateur de particules (100) ; une buse extérieure (212) adaptée pour être reliée de manière séparable à l'élément de liaison (211) de façon à être reliée de manière fixe à l'ouverture de surface supérieure (222) de la chambre optique (220) au niveau de son extrémité inférieure ; un tube d'insertion (213) disposé à l'intérieur de l'élément de liaison (211) et à l'intérieur de la buse extérieure (212), et qui possède un orifice d'insertion dans lequel est placé l'orifice d'évacuation de particules (123) qui s'étend de manière saillante depuis l'extrémité de la face inférieure de la buse de second étage (122) ; une buse intérieure (214) reliée de manière fixe à l'intérieur du tube d'insertion (213) ; un embout de buse (215) relié de manière fixe à l'extrémité de la buse extérieure (212) ; et un élément d'introduction d'air (216) disposé sur l'extérieur de la buse extérieure (212) afin d'y introduire de l'air propre depuis l'extérieur.

5. Dispositif de détection de fluorescence en temps réel selon la revendication 4, dans lequel la partie d'admission (210) comprend en outre un premier moyen de détermination de position destiné à déterminer la position de la buse intérieure (214) lorsque la buse intérieure (214) se trouve dans l'élément de liaison (211) et la buse extérieure (212), le premier moyen de détermination de position ayant une rainure concave (213b) formée sur un côté d'un élément de bague (213a) monté le long de la périphérie extérieure du tube d'insertion (213) et une broche (212a) insérée de manière fixe dans un orifice de broche formé sur un côté de la périphérie supérieure de la buse extérieure (212) de façon à être insérée dans la rainure concave (213b).

6. Dispositif de détection de fluorescence en temps réel selon la revendication 4, dans lequel la partie d'admission (210) comprend en outre un second moyen de détermination de position destiné à déterminer la position de la partie d'admission (210) elle-même lorsque la partie d'admission (210) est montée sur la chambre optique (220), le second moyen de détermination de position ayant une rainure concave (212d) formée sur un côté d'une saillie arrondie (212c) formée sur la périphérie extérieure de la partie d'extrémité inférieure de la buse extérieure (212), et une broche (221a) disposée sur un côté de l'ouverture (222) formée sur la surface supérieure de la chambre optique (220) de façon à être insérée dans la rainure concave (212d).

7. Dispositif de détection de fluorescence en temps réel selon la revendication 6, dans lequel la partie d'admission (210) est reliée de manière fixe à l'ouverture de surface supérieure (222) de la chambre optique (220) à l'aide d'un raccord vissé entre un élément à écrou (217) dont la périphérie intérieure supérieure est appuyée contre la saillie arrondie (212c) formée sur la périphérie extérieure de la partie d'extrémité inférieure de la buse extérieure (212a), et d'un filetage à vis formé sur la périphérie intérieure de celle-ci et d'un élément de liaison (221) qui sort vers le haut depuis le bord de l'ouverture de surface supérieure (222) de la chambre optique (220) et qui possède un filetage à vis formé sur sa périphérie extérieure.

8. Dispositif de détection de fluorescence en temps réel selon la revendication 7, dans lequel la broche (221a) est insérée de manière fixe dans un orifice de broche formé sur un côté de la périphérie de surface supérieure de l'élément de liaison (221).

9. Dispositif de détection de fluorescence en temps réel selon la revendication 4, dans lequel, afin de permettre à la partie d'admission (210) d'être alignée avec l'emplacement de montage sur la chambre optique (220), sans aucune excentricité dans la direction horizontale par rapport à l'emplacement de montage déterminé sur la chambre optique (220) lorsque la partie d'admission (210) est montée contre celle-ci après la séparation de la chambre optique (220), la buse extérieure (212) de la partie d'admission (210) présente une partie inclinée anti-excentricité (212e) formée sur sa partie d'extrémité inférieure, la partie inclinée anti-excentricité (212e) ayant une structure inversement conique de façon à être inclinée vers l'ouverture de surface supérieure (222) de la chambre optique (220), et l'ouverture de surface supérieure (222) de la chambre optique (220) présente une surface inclinée (222a) formée sur l'extrémité inférieure de sa périphérie intérieure de façon à être amenée en contact étroit avec la partie inclinée anti-excentricité (212e).

10. Dispositif de détection de fluorescence en temps réel selon la revendication 9, dans lequel les angles d'inclinaison de la partie inclinée anti-excentricité (212e) et de la surface inclinée (222a) sont identiques, et sont compris entre 25° et 35° par rapport à l'axe central de la partie d'admission (210).

11. Dispositif de détection de fluorescence en temps réel selon la revendication 1, dans lequel la buse d'évacuation (241) de la partie d'évacuation de particules (240) possède une structure effilée dont le diamètre diminue vers sa partie d'extrémité avant, tout en étant disposée pour faire face à l'embout de buse (215) relié à l'extrémité de la buse extérieure (212) de la partie d'admission (210) dans un état d'espacement, selon une distance donnée, de l'embout de buse (215).

12. Dispositif de détection de fluorescence en temps réel selon la revendication 1, dans lequel la partie de mise en forme de faisceau (250) comprend : une partie de source de lumière (251) qui possède un corps (251a) qui présente un espace intérieur ouvert au niveau de ses deux parties d'extrémité afin de monter une lentille asphérique (254) à l'intérieur, et une plaque de recouvrement (251b) qui possède une fiche dans laquelle une diode laser (253) est intégrée au niveau de la partie avant du corps (251a) ; et une partie de mise en forme et d'ajustement de faisceau (252) reliée de manière séparable à la partie de source de lumière (251) et qui possède un premier groupe de lentilles (255), un second groupe de lentilles (256) et une fenêtre (257) prévus dans cet ordre à l'intérieur, le premier groupe de lentilles (255) étant adapté pour ajuster la taille dans la direction longitudinale du faisceau laser généré par la diode laser (253) et qui passe par la lentille asphérique (254), et le second groupe de lentilles (256) étant adapté pour ajuster la taille dans la direction transversale du faisceau laser qui passe par le premier groupe de lentilles (255), et la partie de mise en forme de faisceau (250) est noircie sur sa surface intérieure.

13. Dispositif de détection de fluorescence en temps réel selon la revendication 12, dans lequel la partie de source de lumière (251) de la partie de mise en forme de faisceau (250) comprend un moyen d'alignement de faisceau laser qui possède une pluralité de boulons hexagonaux (251c) insérés dans une pluralité de premiers trous taraudés traversants (251g1) formés en étant espacés les uns des autres sur la périphérie de bord intérieur de la plaque de recouvrement (251b), et une pluralité de trous taraudés non-traversants (251h) formés sur le corps (251a) de façon à correspondre aux premiers trous taraudés traversants (251g1), chaque boulon hexagonal (251c) ayant un ressort (251d) monté sur sa périphérie extérieure afin d'ajuster la force de pressurisation de la plaque de recouvrement (251b) vers le corps (251a), et une pluralité de boulons sans tête (251e) insérés dans une pluralité de seconds trous taraudés traversants (251g2) formés entre les premiers trous taraudés traversants voisins (251g1) formés sur la plaque de recouvrement (251b) afin d'ajuster la force de poussée de la plaque de recouvrement (251b) depuis le corps (251a) selon son degré de fixation.

14. Dispositif de détection de fluorescence en temps réel selon la revendication 13, dans lequel chaque boulon sans tête (251e) est inséré dans une bague (251i) fixée sur le second trou taraudé traversant (251g2) et possède une bille insérée dans son extrémité afin de réaliser un écart de couple, la bague (251i) ayant une rainure d'incision (251i1) formée sur une surface latérale dans une direction longitudinale de celle-ci.

15. Dispositif de détection de fluorescence en temps réel selon la revendication 13, dans lequel le moyen d'alignement de faisceau laser comprend en outre des boulons de fixation (251f1, 251f2 et 251f3) insérés dans trois groupes de troisièmes trous taraudés traversants (251g3) espacés les uns des autres le long de la périphérie de la plaque de recouvrement (251a) de façon à communiquer avec les seconds trous taraudés traversants (251g2), afin de maintenir et fixer les bagues (251i) dans lesquelles les boulons sans tête (251e) sont insérés.

16. Dispositif de détection de fluorescence en temps réel selon la revendication 15, dans lequel chaque groupe de troisièmes trous taraudés traversants (251g3) est formé de trois trous taraudés traversants de forme triangulaire.

17. Dispositif de détection de fluorescence en temps réel selon la revendication 13, dans lequel la partie de source de lumière (251) de la partie de mise en forme de faisceau (250) comprend en outre : une partie cylindrique (251j) formée sur une surface latérale de la plaque de recouvrement (251b) de façon à sortir à l'horizontale vers le corps (251a), et qui possède un espace intérieur par lequel passe le faisceau laser L généré par la diode laser (253) ; une plaque de fixation (251o) disposée à l'intérieur de la partie d'extrémité avant du corps (251a) de façon à avoir une partie ouverte formée pour contenir la partie cylindrique (251j) en entourant la périphérie extérieure de la partie cylindrique (251j), la plaque de fixation (251o) étant fixée sur le corps (251a) à l'aide d'un boulon de fixation (251p) ; et une partie de fixation de lentille asphérique (2511) montée sur l'espace intérieur de la partie cylindrique (251j) et ayant ses deux parties d'extrémité ouvertes de façon à fixer la lentille asphérique (254) sur son espace intérieur.

18. Dispositif de détection de fluorescence en temps réel selon la revendication 17, dans lequel la partie cylindrique (251j) possède un quatrième trou taraudé traversant (251g4) formé sur sa surface supérieure de façon à communiquer avec son espace intérieur, le quatrième trou taraudé traversant (251g4) ayant une vis de fixation (251k) insérée à l'intérieur, la partie de fixation de lentille asphérique (2511) ayant une rainure d'alignement (251m) formée sur la surface supérieure de sa partie d'extrémité arrière afin d'aligner la position de la partie de fixation de lentille asphérique (251l), et le corps (251a) de la partie de source de lumière (251) possède un orifice d'alignement (251n) formé sur un côté de sa surface supérieure de façon à communiquer avec l'espace intérieur du corps (251a) et à correspondre avec la vis de fixation (251k) insérée dans le quatrième trou taraudé traversant (251g4) de la partie cylindrique (251j) et la position de la rainure d'alignement (251m) de la partie de fixation de lentille asphérique (251l), de façon à permettre à la position de l'arbre central de la partie cylindrique (251j) et à la position de l'arbre central de la partie de fixation de lentille asphérique (2511) d'être ajustées depuis l'extérieur.

19. Dispositif de détection de fluorescence en temps réel selon la revendication 1, dans lequel la partie de décharge de faisceau (260) comprend : un corps (261) fixé sur la surface arrière de la chambre optique (220) ; un ensemble de lentille focale (291) adapté pour être inséré dans un orifice traversant d'insertion (262a) formé sur le centre du corps (261) ; un ensemble d'orifice de broche (296) fixé sur une partie concave formée sur la surface arrière du corps (261) de façon à permettre à l'axe optique de l'ensemble de lentille focale (291) de passer par un orifice de broche (293a) ; un boîtier (263) fixé sur l'extrémité arrière de l'ensemble d'orifice de broche (296) ; et un détecteur de sortie de source de lumière (264) fixé sur l'extrémité arrière du boîtier (263) de façon à être incliné à un angle donné par rapport à la surface de référence perpendiculaire à l'axe optique de l'ensemble de lentille focale (291), l'ensemble d'orifice de broche (296) et le boîtier (264) ayant une cavité formée sur ses parties centrales afin de permettre au faisceau laser L qui est passé par l'orifice de broche (293a) de l'ensemble d'orifice de broche (296) d'être étendu en largeur lorsqu'il se rapproche du détecteur de sortie de source de lumière (264).

20. Dispositif de détection de fluorescence en temps réel selon la revendication 1, dans lequel le réflecteur asphérique (232) est disposé sur l'ouverture (226) formée sur la surface de gauche de la chambre optique (220), et l'ouverture (226) est fermée par une plaque d'étanchéité séparable (280).

21. Dispositif de détection de fluorescence en temps réel selon la revendication 1, dans lequel le détecteur de lumière de diffusion (274) possède une photodiode en avalanche (APD) et un amplificateur.

22. Dispositif de détection de fluorescence en temps réel selon la revendication 1, dans lequel l'un des détecteurs de fluorescence (272) et (273) est adapté pour détecter un signal de longueur d'onde longue, et l'autre est adapté pour détecter un signal de longueur d'onde courte, chaque détecteur de fluorescence ayant un filtre optique (275) monté sur sa surface avant afin de couper la lumière diffusée et de faire passer la fluorescence induite à l'intérieur.

23. Dispositif de détection de fluorescence en temps réel selon la revendication 12, dans lequel les détecteurs de fluorescence (272) et (273) sont composés de tubes photomultiplicateurs (PMT).
